# EUROPEAN PATENT APPLICATION

(11) **EP 2 044 939 A1**
(43) Date of publication of application: **08.04.2009**
(21) Application number: 07790463.9
(22) Date of filing: 29.06.2007
(51) Int. Cl.: A61K 31/47, A61K 31/4545, A61K 31/4709, A61P 1/16, A61P 43/00, C07D 215/20, C07D 215/48, C07D 401/12, C07D 417/12

(54) **THERAPEUTIC AGENT FOR LIVER FIBROSIS**

(30) Priority: 29.06.2006 US 817872 P
(71) Applicant: Eisai R&D Management Co., Ltd., Tokyo 112-8088 (JP)
(72) Inventor: YOKOHAMA, Hiromitsu, Tokyo 112-8088 (JP); MATSUOKA, Toshiyuki, Tokyo 112-8088 (JP)
(74) Representative: Woods, Geoffrey Corlett
(86) International application number: PCT/JP2007/063525
(87) International publication number: WO 2008/001956

(57) **Abstract**

The object is to provide a therapeutic agent for hepatic fibrosis and a method for treatment of hepatic fibrosis. 4-(3-chloro-4-(cyclopropylaminocarbonyl)aminophenoxy)-7-methoxy-6-quinolinecarbox amide or an analogue thereof can prevent the fibrillation in the liver, and therefore can be used as a therapeutic agent for hepatic fibrosis or in the method for treatment of hepatic fibrosis.

## Description

### FIELD OF THE INVENTION

The present invention relates to a therapeutic agent for hepatic fibrosis comprising a compound represented by General Formula (I), a pharmacologically acceptable salt thereof, or a solvate of said compound or said salt (hereinafter, also referred to as a "compound of the invention"), to a method for treating hepatic fibrosis comprising administering an effective amount of the compound of the invention to a patient, use of the compound of the invention for producing the therapeutic agent for hepatic fibrosis and to the compound of the invention for the therapeutic agent for hepatic fibrosis.

Furthermore, the present invention relates to a discoidin domain receptor family, member 2 (DDR2) inhibitor.

### BACKGROUND OF THE INVENTION

Hepatic fibrosis is a condition of proliferation of collagen-based collagen fibers in the liver resulting from variously-caused hepatocellular injury. Hepatic fibrosis may be caused by various causes of liver damage such as infection (hepatitis B virus, hepatitis C virus, etc.), alcohol abuse, autoimmune disease, diabetes and bile duct obstruction. Progression of fibrosis of the liver is known to lead to cirrhosis and even hepatic carcinoma at high rates.

Accordingly, inhibition of hepatic fibrosis has been an important issue in preventing cirrhosis and hepatic carcinoma.

So far, however, no effective therapeutic agent for hepatic fibrosis has been developed.

In a hepatic fibrosis models evoked by carbon tetrachloride administration or obstruction of bile duct of rats, increase in expressions of discoidin domain receptor family, member 2 (hereinafter, also referred to as "DDR2") at gene and protein levels as well as increase in autophosphorylation of DDR2 have been reported⁽¹⁾.

Expression of a DDR2 mutant obtained by mutating or deleting the kinase domain of DDR2 in a rat hepatic stellate cells have been reported to inhibit proliferation of said cells⁽¹⁾.

DDR2 has also been reported to play a significant role in hepatic fibrosis⁽¹⁻³⁾.

Thus, it has been suggested that inhibition of DDR2 activation can inhibit hepatic fibrosis and that a DDR2 inhibitor is effective against hepatic fibrosis.

4-(3-chloro-4-(cyclopropylaminocarbonyl)aminophenoxy)-7-methoxy-6-quinoli necarboxamide or an analog thereof is known as an angiogenesis inhibitory⁽⁴⁻⁶⁾. There has been, however, no report as to whether 4-(3-chloro-4-(cyclopropylaminocarbonyl)aminophenoxy)-7-methoxy-6-quinolinecarbox amide or an analog thereof has DDR2 kinase inhibitory activity.

### References

(1) The Journal of Clinical Investigation, 108(9), 1369-1378, 2001
(2) Biochemistry, 41(37), 11091-11098, 2002
(3) Cellular Signaling, 18, 1108-1116, 2006
(4) International Publication No. 02/32872
(5) International Publication No. 2004/080462
(6) International Publication No. 2005/063713

### DISCLOSURE OF THE INVENTION

The present invention was achieved regarding the circumstances described above and the problems to be solved by the invention are to provide a therapeutic agent for hepatic fibrosis and a method for treating hepatic fibrosis.

In order to solve the above problems, we have gone through keen examination, as a result of which we found that the compound represented by General Formula (I), a pharmacologically acceptable salt thereof, or a solvate of said compound or said salt has DDR2 kinase inhibitory activity as well as hepatic fibrosis inhibitory action.

Thus, the present invention relates to the followings.
(1) A therapeutic agent for hepatic fibrosis comprising a compound represented by General Formula (I), a pharmacologically acceptable salt thereof, or a solvate of said compound or said salt.
(2) A method for treating hepatic fibrosis comprising administering an effective amount of a compound represented by General Formula (I), a pharmacologically acceptable salt thereof, or a solvate of said compound or said salt to a patient.
(3) Use of a compound represented by General Formula (I), a pharmacologically acceptable salt thereof, or a solvate of said compound or said salt for producing a therapeutic agent for hepatic fibrosis.
(4) A compound represented by General Formula (I), a pharmacologically acceptable salt thereof, or a solvate of said compound or said salt for a therapeutic agent for hepatic fibrosis.
(5) A DDR2 inhibitor comprising a compound represented by General Formula (I), a pharmacologically acceptable salt thereof, or a solvate of said compound or said salt.

The compound represented by General Formula (I), a pharmacologically acceptable salt thereof, or a solvate of said compound or said salt is as follows.

A compound represented by General Formula (I) 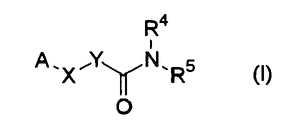 [wherein, A represents a group represented by any one of the following formulae 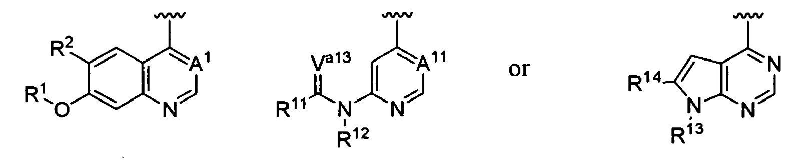
(wherein, R¹ represents a group represented by Formula V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
A¹ represents an optionally substituted carbon atom or nitrogen atom;
R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group or an optionally substituted mono-C₁₋₆ alkylamino group; R¹² represents a hydrogen atom or an optionally substituted C₁₋₆ alkyl group;
V^{a13} represents an oxygen atom or a sulfur atom;
A¹¹ represents an optionally substituted carbon atom or nitrogen atom;
R¹³ represents a hydrogen atom, an optionally substituted C₁₋₆ alkyl group or an optionally substituted C₃₋₈ cycloalkyl group;
R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group));
X represents an oxygen atom or a sulfur atom;
Y represents a group represented by any one of the following formulae 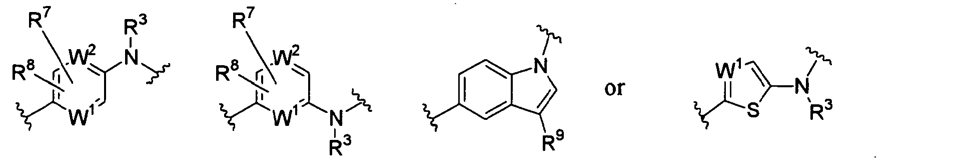
   (wherein, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
   R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group);
   R⁹ represents a hydrogen atom, a halogen atom or an optionally substituted C₁₋₆ alkyl group;
   W¹ and W² each independently represent an optionally substituted carbon atom or nitrogen atom);
   R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
   R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group], a pharmacologically acceptable salt thereof or a solvate thereof.

The present invention further relates to the followings.
(6) A therapeutic agent for hepatic fibrosis comprising 4-(3-chloro-4-(cyclopropylaminocarbonyl)aminophenoxy)-7-methoxy-6-quinolinecarbox amide, a pharmacologically acceptable salt thereof, or a solvate of said compound or said salt.
(7) A method for treating hepatic fibrosis comprising administering an effective amount of 4-(3-chloro-4-(cyclopropylaminocarbonyl)aminophenoxy)-7-methoxy-6-quinolinecarbox amide, a pharmacologically acceptable salt thereof, or a solvate of said compound or said salt to a patient.
(8) Use of 4-(3-chloro-4-(cyclopropylaminocarbonyl)aminophenoxy)-7-methoxy-6-quinolinecarbox amide, a pharmacologically acceptable salt thereof, or a solvate of said compound or said salt for producing a therapeutic agent for hepatic fibrosis.
(9) 4-(3-chloro-4-(cyclopropylaminocarbonyl)aminophenoxy)-7-methoxy-6-quinolinecarbox amide, a pharmacologically acceptable salt thereof, or a solvate of said compound or said salt for a therapeutic agent for hepatic fibrosis.
(10) A DDR2 inhibitor comprising 4-(3-chloro-4-(cyclopropylaminocarbonyl)aminophenoxy)-7-methoxy-6-quinolinecarbox amide, a pharmacologically acceptable salt thereof, or a solvate of said compound or said salt.

The present invention provides a therapeutic agent for hepatic fibrosis comprising a compound of the invention, a method for treating hepatic fibrosis, use of the compound of the invention for producing the therapeutic agent for hepatic fibrosis and the compound of the invention for the therapeutic agent for hepatic fibrosis.

The present invention also provides a DDR2 inhibitor.

### BREIF DESCRIPTION OF THE DRAWING

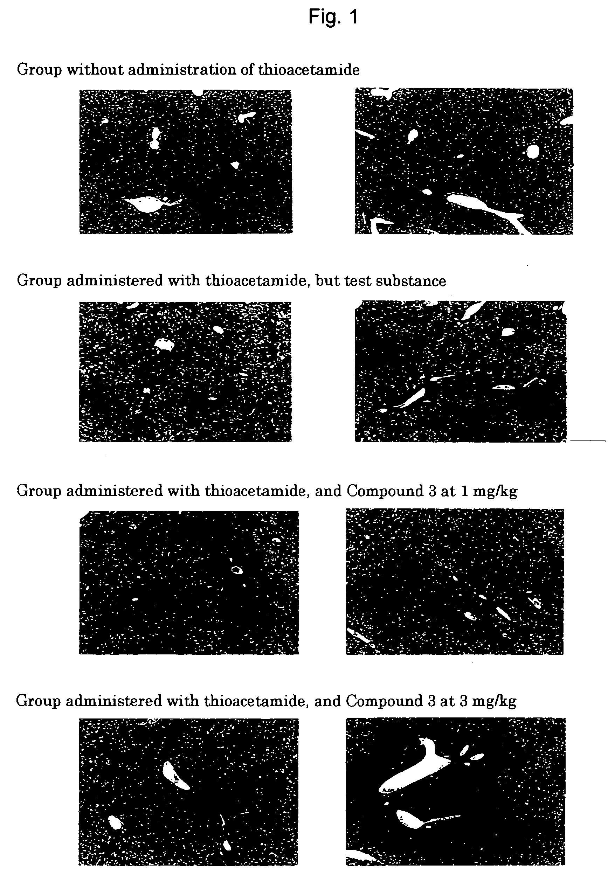

Figure 1 shows representative results from staining liver tissue sections for a group without administration ofthioacetamide, a group administered with thioacetamide but a test substance, a group administered with Compound 3 at 1 mg/kg and a group administered with Compound 3 at 3 mg/kg.

### BEST MODES FOR CARRYING OUT THE INVENTION

Hereinafter, embodiments of the present invention will be described. The following embodiments are provided for illustrating the present invention, and the present invention is not intended thereto. The present invention may be carried out in various embodiments without departing from the scope of the invention.

The present specification incorporates the content of specification of US provisional patent application No. 60/817, 872 (filed on June 29, 2006) based on which the present application claims priority. The documents, laid-open patent publications, patent publications and other patent documents cited herein are incorporated herein by reference.

### 1. Therapeutic agent and method of the invention

### (1) DDR2

According to the present invention, DDR2 stands for discoidin domain receptor family, member 2, which includes, for example, a polypeptide comprising the amino acid sequence represented by SEQ ID NO:2 (GenBank Accession No: NM_001014796, NM_006182). A polypeptide comprising the amino acid sequence represented by SEQ ID NO:2 is encoded, for example, by a polynucleotide comprising a nucleotide sequence represented by SEQ ID NO: 1. Usually, processing yields mature form of the polypeptide consisting of the amino acid sequence represented by SEQ ID NO:2.

### (2) Compound of the invention.

Herein, a "halogen atom" refers to a fluorine atom, a chlorine atom, a bromine atom or an iodine atom.

Preferable examples of a "halogen atom" include a fluorine atom and a chlorine atom.

Herein, a "C₁₋₆ alkyl group" refers to a linear or branched alkyl group with a carbon number of 1-6, specific examples including a methyl group, an ethyl group, a 1-propyl group (n-propyl group), a 2-propyl group (i-propyl group), a 2-methyl-1-propyl group (i-butyl group), a 2-methyl-2-propyl group (t-butyl group), a 1-butyl group (n-butyl group), a 2-butyl group (s-butyl group), a 1-pentyl group, a 2-pentyl group, a 3-pentyl group, a 2-methyl-1-butyl group, a 3-methyl-1-butyl group, a 2-methyl-2-butyl group, a 3-methyl-2-butyl group, a 2,2-dimethyl-1-propyl group, a 1-hexyl group, a 2- hexyl group, a 3- hexyl group, a 2-methyl-1-pentyl group, a 3-methyl-1-pentyl group, a 4-methyl-1-pentyl group, a 2-methyl-2-pentyl group, a 3-methyl-2-pentyl group, a 4-methyl-2-pentyl group, a 2-methyl-3-pentyl group, a 3-methyl-3-pentyl group, a 2,3-dimethyl-1-butyl group, a 3,3-dimethyl-1-butyl group, a 2,2-dimethyl-1-butyl group, a 2-ethyl-1-butyl group, a 3,3-dimethyl-2-butyl group and a 2,3-dimethyl-2-butyl group

Preferable examples of a "C₁₋₆ alkyl group" include a methyl group, an ethyl group, a 1-propyl group, a 2-propyl group, a 2-methyl-1-propyl group, a 2-methyl-2-propyl group, a 1-butyl group and a 2-butyl group.

Herein, a "C₁₋₆ alkylene group" refers to a divalent group derived from the "C₁₋₆ alkyl group" defined above by removing any one hydrogen atom therefrom, specific examples including a methylene group, a 1,2-ethylene group, a 1,1-ethylene group, a 1,3-propylene group, a tetramethylene group, a pentamethylene group and a hexamethylene group.

Herein, a "C₂₋₆ alkenyl group" refers to a linear or branched alkenyl group having one double bond and a carbon number of 2-6, specific examples including an ethenyl group (vinyl group), a 1-propenyl group, a 2-propenyl group (allyl group), a 1-butenyl group, a 2-butenyl group, a 3-butenyl group, a pentenyl group and a hexenyl group.

Herein, a "C₂₋₆ alkynyl group" refers to a linear or branched alkynyl group having one triple bond and a carbon number of 2-6, specific examples including an ethinyl group, a 1-propynyl group, a 2-propynyl group, a 1-butynyl group, a 2-butynyl group, a 3-butynyl group, a pentynyl group and a hexynyl group.

Herein, a "C₃₋₈ cycloalkyl group" refers to a monocyclic or bicyclic saturated aliphatic hydrocarbon group with a carbon number of 3-8, specific examples including a cyclopropyl group, a cyclobutyl group, a cyclopentyl group, a cyclohexyl group, a cycloheptyl group, a cyclooctyl group, a bicyclo[2. 1. 0]pentyl group, a bicyclo[3. 1. 0]hexyl group, a bicyclo[2. 1. 1]hexyl group, a bicyclo[4. 1. 0]heptyl group, a bicyclo[2. 2. 1]heptyl group (norbornyl group), a bicyclo[3. 3. 0]octyl group, a bicyclo[3. 2. 1]octyl group and a bicyclo[2. 2. 2]octyl group.

Preferable examples of a "C₃₋₈ cycloalkyl group" include a cyclopropyl group, a cyclobutyl group and a cyclopentyl group.

Herein, a "C₆₋₁₀ aryl group" refers to an aromatic hydrocarbon cyclic group with a carbon number of 6-10, specific examples including a phenyl group, a 1-naphthyl group, a 2-naphthyl group, an indenyl group and an azulenyl group.

A preferable example of a "C₆₋₁₀ aryl group" includes a phenyl group.

Herein, a "heteroatom" refers to a nitrogen atom, an oxygen atom or a sulfur atom.

Herein, a "5-10-membered heteroaryl group" refers to an aromatic cyclic group having 5-10 atoms forming the ring and 1-5 heteroatoms included in the atoms forming the ring, specific examples including a furyl group, a thienyl group, a pyrrolyl group, an imidazolyl group, a triazolyl group, a tetrazolyl group, a thiazolyl group, a pyrazolyl group, an oxazolyl group, an isoxazolyl group, an isothiazolyl group, a furazanyl group, a thiadiazolyl group, an oxadiazolyl group, a pyridyl group, a pyrazinyl group, a pyridazinyl group, a pyrimidinyl group, a triazinyl group, a purinyl group, a pteridinyl group, a quinolyl group, an isoquinolyl group, a naphthyridinyl group, a quinoxalinyl group, a cinnolinyl group, a quinazolinyl group, a phthalazinyl group, an imidazopyridyl group, an imidazothiazolyl group, an imidazoxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzoxadiazolyl group, a pyridopyrimidinyl group, a benzofuryl group, a benzothienyl group and a thienofuryl group.

Preferable examples of a "5-10-membered heteroaryl group" include a furyl group, a thienyl group, a pyrrolyl group, an imidazolyl group, a thiazolyl group, a pyrazolyl group, an oxazolyl group, an isoxazolyl group, an isothiazolyl group, a pyridyl group and a pyrimidinyl group.

Herein, a "3-10-membered nonaromatic heterocyclic group":
(a) has 3-10 atoms forming the ring;
(b) has 1-2 heteroatoms included in the atoms forming the ring;

### (3) may include 1-2 double bonds in the ring;

(d) may have 1-3 carbonyl groups, sulfinyl groups or sulfonyl groups in the ring; and
(e) is a nonaromatic monocyclic or bicyclic group where when a nitrogen atom is included in the atoms forming the ring, the nitrogen atom may have a binding hand.

Specific examples include an aziridinyl group, an azetidinyl group, a pyrrolidinyl group, a piperidinyl group, an azepanyl group, an azocanyl group, a piperazinyl group, a diazepanyl group, a diazocanyl group, a diazabicyclo[2. 2. 1]heptyl group, a morpholinyl group, a thiomorpholinyl group, a 1,1-dioxothiomorpholinyl group, an oxiranyl group, an oxetanyl group, a tetrahydrofuryl group, a dioxoranyl group, a tetrahydropyranyl group, a dioxanyl group, a tetrahydrothienyl group, a tetrahydrothiopyranyl group, an oxazolidinyl group and a thiazolidinyl group.

Preferable examples of a "3-10-membered nonaromatic heterocyclic group" include an aziridinyl group, an azetidinyl group, a pyrrolidinyl group, a piperidinyl group, an azepanyl group, a piperazinyl group, a diazepanyl group, a morpholinyl group, a thiomorpholinyl group, a 1,1-dioxothiomorpholinyl group, a tetrahydrofuryl group and a tetrahydropyranyl group.

Herein, a "C₁₋₆ alkoxy group" refers to a group in which an oxygen atom is bound to the terminal of a "C₁₋₆ alkyl group" defined above, specific examples including a methoxy group, an ethoxy group, a 1-propoxy group (n-propoxy group), a 2-propoxy group (i-propoxy group), a 2-methyl-1-propoxy group (i-butoxy group), a 2-methyl-2-propoxy group (t-butoxy group), a 1-butoxy group (n-butoxy group), a 2-butoxy group (s-butoxy group), a 1-pentyloxy group, a 2-pentyloxy group, a 3-pentyloxy group, a 2-methyl-1-butoxy group, a 3-methyl-1-butoxy group, a 2-methyl-2-butoxy group, a 3-methyl-2-butoxy group, a 2,2-dimethyl-1-propoxy group, a 1-hexyloxy group, a 2-hexyloxy group, a 3-hexyloxy group, a 2-methyl-1-pentyloxy group, a 3-methyl-1-pentyloxy group, a 4-methyl-1-pentyloxy group, a 2-methyl-2-pentyloxy group, a 3-methyl-2-pentyloxy group, a 4-methyl-2-pentyloxy group, a 2-methyl-3-pentyloxy group, a 3-methyl-3-pentyloxy group, a 2,3-dimethyl-1-butoxy group, a 3,3-dimethyl-1-butoxy group, a 2,2-dimethyl-1-butoxy group, a 2-ethyl-1-butoxy group, a 3,3-dimethyl-2-butoxy group and a 2,3-dimethyl-2-butoxy group.

Preferable examples of a "C₁₋₆ alkoxy group" include a methoxy group, an ethoxy group, a 1-propoxy group, a 2-propoxy group, a 2-methyl-1-propoxy group, a 2-methyl-2-propoxy group, a 1-butoxy group and a 2-butoxy group.

Herein, a "C₁₋₆ alkylthio group" refers to group in which a sulfur atom is bound to the terminal of a "C₁₋₆ alkyl group" defined above, specific examples including a methylthio group, an ethylthio group, a 1-propylthio group (n-propylthio group), a 2-propylthio group (i-propylthio group), a 2-methyl-1-propylthio group (i-butylthio group), a 2-methyl-2-propylthio group (t-butylthio group), a 1-butylthio group (n-butylthio group), a 2-butylthio group (s-butylthio group), a 1-pentylthio group, a 2-pentylthio group, a 3-pentylthio group, a 2-methyl-1-butylthio group, a 3-methyl-1-butylthio group, a 2-methyl-2-butylthio group, a 3-methyl-2-butylthio group, a 2,2-dimethyl-1-propylthio group, a 1-hexylthio group, a 2-hexylthio group, a 3-hexylthio group, a 2-methyl-1-pentylthio group, a 3-methyl-1-pentylthio group, a 4-methyl-1-pentylthio group, a 2-methyl-2-pentylthio group, a 3-methyl-2-pentylthio group, a 4-methyl-2-pentylthio group, a 2-methyl-3-pentylthio group, a 3-methyl-3-pentylthio group, a 2,3-dimethyl-1-butylthio group, a 3,3-dimethyl-1-butylthio group, a 2,2-dimethyl-1-butylthio group, a 2-ethyl-1-butylthio group, a 3,3-dimethyl-2-butylthio group and a 2,3-dimethyl-2-butylthio group.

Preferable examples of a "C₁₋₆ alkylthio group" include a methylthio group, an ethylthio group, a 1-propylthio group (n-propylthio group), a 2-propylthio group (i-propylthio group), a 2-methyl-1-propylthio group (i-butylthio group), a 2-methyl-2-propylthio group (t-butylthio group), a 1-butylthio group (n-butylthio group) and a 2-butylthio group (s-butylthio group).

Herein, a "C₃₋₈ cycloalkoxy group" refers to a group in which an oxygen atom is bound to the terminal of a "C₃₋₈ cycloalkyl group" defined above, specific examples including a cyclopropoxy group, a cyclobutoxy group, a cyclopentyloxy group, a cyclohexyloxy group, a cycloheptyloxy group, a cyclooctyloxy group, a bicyclo[2. 1. 0]pentyloxy group, a bicyclo[3. 1. 0]hexyloxy group, a bicyclo[2. 1. 1]hexyloxy group, a bicyclo[4. 1. 0]heptyloxy group, a bicyclo[2. 2. 1]heptyloxy group (norbornyloxy group), a bicyclo[3. 3. 0]octyloxy group, a bicyclo[3. 2. 1]octyloxy group and a bicyclo[2. 2. 2]octyloxy group.

Preferable examples of a "C₃₋₈ cycloalkoxy group" include a cyclopropoxy group, a cyclobutoxy group and a cyclopentyloxy group.

Herein, a "mono-C₁₋₆ alkylamino group" refers to a group in which a hydrogen atom in an amino group is substituted with a "C₁₋₆ alkyl group" defined above, specific examples including a methylamino group, an ethylamino group, a 1-propylamino group (n-propylamino group), a 2-propylamino group (i-propylamino group), a 2-methyl-1-propylamino group (i-butylamino group), a 2-methyl-2-propylamino group (t-butylamino group), a 1-butylamino group (n-butylamino group), a 2-butylamino group (s-butylamino group), a 1-pentylamino group, a 2-pentylamino group, a 3-pentylamino group, a 2-methyl-1-butylamino group, a 3-methyl-1-butylamino group, a 2-methyl-2-butylamino group, a 3-methyl-2-butylamino group, a 2,2-dimethyl-1-propylamino group, a 1-hexylamino group, a 2-hexylamino group, a 3-hexylamino group, a 2-methyl-1-pentylamino group, a 3-methyl-1-pentylamino group, a 4-methyl-1-pentylamino group, a 2-methyl-2-pentylamino group, a 3-methyl-2-pentylamino group, a 4-methyl-2-pentylamino group, a 2-methyl-3-pentylamino group, a 3-methyl-3-pentylamino group, a 2,3-dimethyl-1-butylamino group, a 3,3-dimethyl-1-butylamino group, a 2,2-dimethyl-1-butylamino group, a 2-ethyl-1-butylamino group, a 3,3-dimethyl-2-butylamino group and a 2,3-dimethyl-2-butylamino group.

Herein, a "di-C₁₋₆ alkylamino group" refers to a group in which two hydrogen atoms in an amino group are substituted with an identical or different "C₁₋₆ alkyl group" defined above, specific examples including a N,N-dimethylamino group, a N,N-diethylamino group, a N,N-di-n-propylamino group, a N,N-di-i-propylamino group, a N,N-di-n-butylamino group, a N,N-di-i-butylamino group, a N,N-di-s-butylamino group, a N,N-di-t-butylamino group, a N-ethyl-N-methylamino group, a N-n-propyl-N-methylamino group, a N-i-propyl-N-methylamino group, a N-n-butyl-N-methylamino group, a N-i-butyl-N-methylamino group, a N-s-butyl-N-methylamino group and a N-t-butyl-N-methylamino group.

Herein, a "C₂₋₇ acyl group" refers to a carbonyl group bound with a "C₁₋₆ alkyl group" defined above, specific examples including an acetyl group, a propionyl group, an isopropionyl group, a butyryl group, an isobutyryl group, a valeryl group, an isovaleryl group and a pivaloyl group.

Herein, a "C₂₋₇ alkoxycarbonyl group" refers to a carbonyl group bound with a "C₁₋₆ alkoxy group" defined above, specific examples including a methoxycarbonyl group, an ethoxycarbonyl group, a 1-propyloxycarbonyl group, a 2-propyloxycarbonyl group and a 2-methyl-2-propoxycarbonyl group.

Herein, "optionally substituted" or "that may have a substituent" means "that may have one or more substituents in any combination at substitutable positions". Specific examples of the substituent includes a halogen atom, a hydroxyl group, a thiol group, a nitro group, a cyano group, a formyl group, a carboxyl group, an amino group, a silyl group, a methanesulfonyl group, a C₁₋₆ alkyl group, a C₂₋₆ alkenyl group, a C₂₋₆ alkynyl group, a C₃₋₈ cycloalkyl group, a C₆₋₁₀ aryl group, a 5-10-membered heteroaryl group, a 3-10-membered nonaromatic heterocyclic group, a C₁₋₆ alkoxy group, a C₁₋₆ alkylthio group, a C₃₋₈ cycloalkoxy group, a mono-C₁₋₆ alkylamino group, a di-C₁₋₆ alkylamino group, a C₂₋₇ acyl group and a C₂₋₇ alkoxycarbonyl group. In this case, the C₁₋₆ alkyl group, the C₂₋₆ alkenyl group, the C₂₋₆ alkynyl group, the C₃₋₈ cycloalkyl group, the C₆₋₁₀ aryl group, the 5-10-membered heteroaryl group, the 3-10-membered nonaromatic heterocyclic group, the C₁₋₆ alkoxy group, the C₁₋₆ alkylthio group, the C₃₋₈ cycloalkoxy group, the mono-C₁₋₆ alkylamino group, the di-C₁₋₆ alkylamino group, the C₂₋₇ acyl group and the C₂₋₇ alkoxycarbonyl group may each independently have 1-3 groups selected from the group consisting of the following substituent groups.

### <Substituent groups>

A halogen atom, a hydroxyl group, a thiol group, a nitro group, a cyano group, a C₁₋₆ alkyl group, a C₃₋₈ cycloalkyl group, a C₂₋₆ alkenyl group, a C₂₋₆ alkynyl group, a C₆₋₁₀ aryl group, a 5-10-membered heteroaryl group, a 3-10-membered nonaromatic heterocyclic group, a C₁₋₆ alkoxy group and a C₁₋₆ alkylthio group.

According to the present invention, a compound represented by General Formula (I) is as follows.

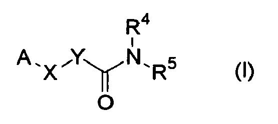

### (i) A

A represents a group represented by any one of the following formulae.

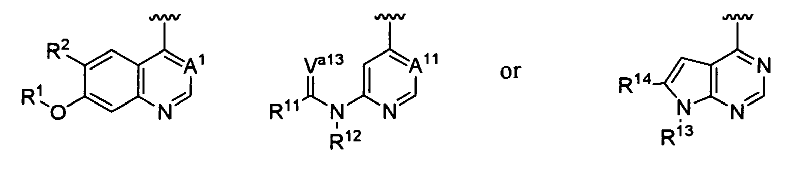

In the formula, R¹ represents a group represented by Formula -V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group).

R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group).

A¹ represents an optionally substituted carbon atom or nitrogen atom.

R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, any optionally substituted 5-10-membered heteroaryl group, an optionally substituted 3-10-membered nonaromatic heterocyclic group or an optionally substituted mono-C₁₋₆ alkylamino group.

R¹² represents a hydrogen atom or an optionally substituted C₁₋₆ alkyl group.

V^{a13} represents an oxygen atom or a sulfur atom.

A¹¹ represents an optionally substituted carbon atom or nitrogen atom.

R¹³ represents a hydrogen atom, an optionally substituted C₁₋₆ alkyl group or an optionally substituted C₃₋₈ cycloalkyl group.

R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group).

### (ii) X

X represents an oxygen atom or a sulfur atom.

### (iii) Y

Y represents a group represented by any one of the following formulae.

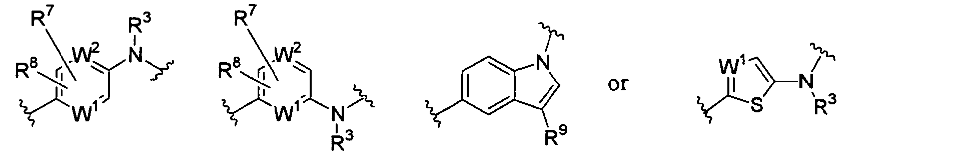

In the formula, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group.

R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group).

R⁹ represents a hydrogen atom, a halogen atom or an optionally substituted C₁₋₆ alkyl group.

W¹ and W² each independently represent an optionally substituted carbon atom or nitrogen atom.

### (iv) R⁴

R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group.

### (v) R⁵

R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group

The compound represented by General Formula (I) can be produced by a known method, for example, by any methods described in International publication No. 02/032872 (WO02/32872), International publication No. 2004/020434 (WO2004/02043) and International publication No. 2005/063713 (WO2005/063713).

According to the present invention, the compound represented by General Formula (I) preferably comprises the compound represented by General Formula (II).

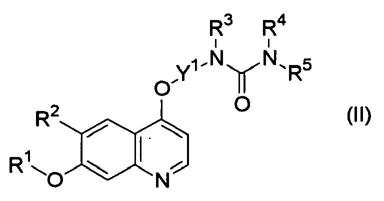

### (i) R¹

R¹ is as defined above.

A preferable example of R¹ includes a C₁₋₆ alkyl group. For example, in the definition of R¹, when V¹ is a C₁₋₆ alkylene group, V² is a single bond and V³ is a hydrogen atom, R¹ is a C₁₋₆ alkyl group provided that R¹ may have a substituent selected from a 3-10-membered nonaromatic heterocyclic group which may have a C₁₋₆ alkyl group, a hydroxyl group, a C₁₋₆ alkoxy group, an amino group, a mono-C₁₋₆ alkylamino group and a di-C₁₋₆ alkylamino group.

More preferable examples of R¹ include a methyl group and a group represented by any one of the following formulae 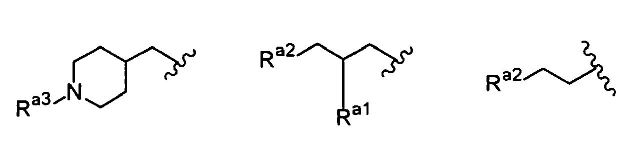 (wherein, R^{a3} represents a methyl group; R^{a1} represents a hydrogen atom or a hydroxyl group; R^{a2} represents a methoxy group, an ethoxy group, a 1-pyrrolidinyl group, a 1-piperidinyl group, a 4-morpholinyl group, a dimethylamino group or a diethylamino group).

Still more preferable examples of R¹ include a methyl group and a 2-methoxyethyl group.

### (ii) R²

R² is as defined above.

Preferable examples of R² include a cyano group and a group represented by Formula -CONV^{a11}V^{a12} (wherein, V^{a11} and V^{a12} have the same meaning as defined above).

More preferable examples of R² include a cyano group and a group represented by Formula -CONHV^{a16} (wherein, V^{a16} represents a hydrogen atom, a C₁₋₆ alkyl group, a C₃₋₈ cycloalkyl group, a C₁₋₆ alkoxy group or a C₃₋₈ cycloalkoxy group, provided that V^{a16} may have a substituent selected from a halogen atom, a cyano group, a hydroxyl group and a C₁₋₆ alkoxy group).

Still more preferable example of R² includes a group represented by Formula -CONHV^{a17} (wherein, V^{a17} represents a hydrogen atom, a C₁₋₆ alkyl group or a C₁₋₆ alkoxy group).

The most preferable example of R² includes a group represented by Formula -CONHV^{a18} (wherein, V^{a18} represents a hydrogen atom, a methyl group or a methoxy group).

### (iii) Y¹

Y¹ represents a group represented by the following formula 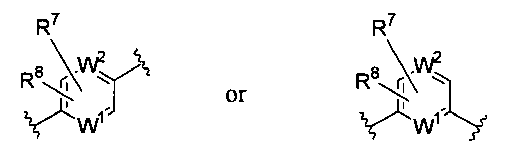 (wherein, R⁷, R⁸, W¹ and W² are as defined as above).

A preferable example of Y¹ includes a group represented by the following formula 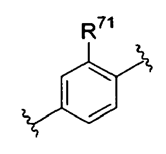 (wherein, R⁷¹ represents a hydrogen atom or a halogen atom).

### (iv) R³ and R⁴

R³ and R⁴ are as defined above.

A preferable example of R³ and R⁴ includes a hydrogen atom.

### (v) R⁵

R⁵ is as defined above.

Preferable examples of R⁵ include a hydrogen atom, a C₁₋₆ alkyl group, a C₃₋₈ cycloalkyl group and a C₆₋₁₀ aryl group (provided that R⁵ may have a substituent selected from a halogen atom and a methanesulfonyl group).

More preferable examples of R⁵ include a methyl group, an ethyl group and a cyclopropyl group.

Moreover, preferable examples of the compound represented by General Formula (I) include:
N-(4-(6-cyano-7-(2-methoxyethoxy)-4-quinolyl)oxy-2-fluorophenyl)-N'-(4-fluor ophenyl)urea;
N-(2-chloro-4-((6-cyano-7-((1-methyl-4-piperidyl)methoxy)-4-quinolyl)oxy)phe nyl)-N'-cyclopropylurea;
N-(4-((6-cyano-7-(((2R)-3-(diethylamino)-2-hydroxypropyl)oxy)-4-quinolyl)oxy )phenyl)-N'-(4-fluorophenyl)urea;
N-(4-((6-cyano-7-(((2R)-2-hydroxy-3-(1-pyrrolidino)propyl)oxy)-4-quinolyl)ox y)phenyl)-N'-(4-fluorophenyl)urea;
4-(3-chloro-4-(cyclopropylaminocarbonyl)aminophenoxy)-7-methoxy-6-quinoli necarboxamide;
4-(3-chloro-4-(cyclopropylaminocarbonyl)aminophenoxy)-7-(2-methoxyethoxy) -6-quinolinecarboxamide;
N6-cyclopropyl-4-(3-chloro-4-(((cyclopropylamino)carbonyl)amino)phenoxy)-7 -methoxy-6-quinolinecarboxamide;
N6-(2-methoxyethyl)-4-(3-chloro-4-(((cyclopropylamino)carbonyl)amino)pheno xy)-7-methoxy-6-quinolinecarboxamide;
N6-(2-fluoroethyl)-4-(3-chloro-4-(((cyclopropylamino)carbonyl)amino)phenoxy )-7-methoxy-6-quinolinecarboxamide;
N6-methoxy-4-(3-chloro-4-(((cyclopropylamino)carbonyl)amino)phenoxy)-7-m ethoxy-6-quinolinecarboxamide;
N6-methyl-4-(3-chloro-4-(((cyclopropylamino)carbonyl)amino)phenoxy)-7-met hoxy-6-quinolinecarboxamide;
N6-ethyl-4-(3-chloro-4-(((cyclopropylamino)carbonyl)amino)phenoxy)-7-metho xy-6-quinolinecarboxamide;
4-(3-fluoro-4-(cyclopropylaminocarbonyl)aminophenoxy)-7-(2-methoxyethoxy) -6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydrox ypropyl)oxy-6-quinolinecarboxamide;
4-(3-chloro-4-(methylaminocarbonyl)aminophenoxy)-7-methoxy-6-quinolinecar boxamide;
4-(3-chloro-4-(ethylaminocarbonyl)aminophenoxy)-7-methoxy-6-quinolinecarbo xamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6 -quinolinecarboxamide;
4-(4-((cyclopropylamino)carbonyl)aminophenoxy)-7-(2-methoxyethoxy)-6-quin olinecarboxamide;
N-(2-fluoro-4-((6-carbamoyl-7-methoxy-4-quinolyl)oxy)phenyl)-N'-cyclopropyl urea;
N6-(2-hydroxyethyl)-4-(3-chloro-4-(((cyclopropylamino)carbonyl)amino)pheno xy)-7-methoxy-6-quinolinecarboxamide;
4-(3-chloro-4-(1-propylaminocarbonyl)aminophenoxy)-7-methoxy-6-quinolinec arboxamide;
4-(3-chloro-4-(cis-2-fluoro-cyclopropylaminocarbonyl)aminophenoxy)-7-metho xy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)e thoxy)-6-quinolinecarboxamide;
4-(3-chloro-4-(2-fluoroethylaminocarbonyl)aminophenoxy)-7-methoxy-6-quinol inecarboxamide;
N6-((2R)tetrahydro-2-furanylmethyl)-4-(3-chloro-4-(((methylamino)carbonyl)a mino)phenoxy)-7-methoxy-6-quinolinecarboxamide;
4-(3-fluoro-4-(ethylaminocarbonyl)aminophenoxy)-7-methoxy-6-quinolinecarbo xamide;
4-(3-chloro-4-(((cyclopropylamino)carbonyl)amino)phenoxy)-7-((2R)-2-hydrox y-3-(1-pyrrolidino)propoxy)-6-quinolinecarboxamide;
N6-methyl-4-(3-chloro-4-(((methylamino)carbonyl)amino)phenoxy)-7-((2R)-3-d iethylamino-2-hydroxypropoxy)-6-quinolinecarboxamide;
N6-methyl-4-(3-chloro-4-(((ethylamino)carbonyl)amino)phenoxy)-7-((2R)-3-die thylamino-2-hydroxypropoxy)-6-quinolinecarboxamide;
N6-methyl-4-(3-chloro-4-(((methylamino)carbonyl)amino)phenoxy)-7-((2R)-2-h ydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hy droxy-3-(1-pyrrolidino)propoxy)-6-quinolinecarboxamide;
N6-methyl-4-(3-chloro-4-(((methylamino)carbonyl)amino)phenoxy)-7-((1-meth yl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopr opylurea;
N-(4-(6-cyano-7-(3-(4-morpholino)propoxy)-4-quinolyl)oxyphenyl)-N'-(3-(meth ylsulfonyl)phenyl)urea;
4-(4-((cyclopropylamino)carbonyl)aminophenoxy)-7-methoxy-6-quinolinecarbo xamide;
4-(3-fluoro-4-((2-fluoroethylamino)carbonyl)aminophenoxy)-7-methoxy-6-quin olinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N '-cyclopropylurea.

More preferable examples of the compound represented by General Formula (I) further include:
4-(3-chloro-4-(cyclopropylaminocarbonyl)aminophenoxy)-7-methoxy-6-quinoli necarboxamide;
4-(3-chloro-4-(ethylaminocarbonyl)aminophenoxy)-7-methoxy-6-quinolinecarbo xamide;
N6-methoxy-4-(3-chloro-4-(((cyclopropylamino)carbonyl)amino)phenoxy)-7-m ethoxy-6-quinolinecarboxamide;
4-(3-chloro-4-(methylaminocarbonyl)aminophenoxy)-7-methoxy-6-quinolinecar boxamide; and
N6-methoxy-4-(3-chloro-4-(((ethylamino)carbonyl)amino)phenoxy)-7-methoxy-6-quinolinecarboxamide.

A still more preferable example of the compound represented by General Formula (I) also includes 4-(3-chloro-4-(cyclopropylaminocarbonyl)aminophenoxy)-7-methoxy-6-quinolinecarbox amide (see Formula (III)).

The most preferable example of the compound of the invention includes methanesulfonate of 4-(3-chloro-4-(cyclopropylaminocarbonyl)aminophenoxy)-7-methoxy-6-quinolinecarbox amide.

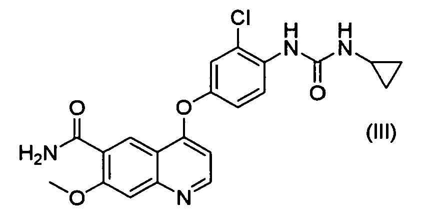

Preferable examples of the compound represented by General Formula (I) also include:
N-(4-methoxyphenyl)-N'-(4-((6,7-dimethoky-4-quinolinyl)oxy)phenyl)urea (Compound 1);
N-n-butyl-N'-(4-((6,7-dimethoxy-4-quinolinyl)oxy)phenyl)urea (Compound 2);
N-(2-thiazole)-N'-(4-((6,7-dimethoxy-4-quinolinyl)oxy)phenyl)urea (Compound 3);
N-(3-carbamoylphenyl)-N'-(4-((6,7-dimethoxy-4-quinolinyl)oxy)phenyl)urea (Compound 4);
N-(3-methylthiophenyl)-N'-(4-((6,7-dimethoxy-4-quinolinyl)oxy)phenyl)urea (Compound 5);
N-(3-methylsulfonylphenyl)-N'-(4-((6,7-dimethoxy-4-quinolinyl)oxy)phenyl)ur ea (Compound 6);
N-(4-(6-cyano-7-(2-methoxyethoxy)-4-quinolyl)oxy-2-fluorophenyl)-N'-(3-met hylsulfonylphenyl)urea (Compound 7);
4-(4-((4-fluoroanilino)carbonyl)amino-3-fluorophenoxy)-7-methoxy-6-quinoline carboxamide (Compound 8);
6,7-dimethoxy-4-(5-(1-(4-fluorophenylcarbamoyl)-indolyl)oxy)quinoline (Compound 9);
4-(3-fluoro-4-((3-methylthiopropylamino)carbonyl)aminophenoxy)-7-methoxy-6-quinolinecarboxamide (Compound 10);
4-(3-chloro-4-(cyclopropylaminocarbonyl)aminophenoxy)-7-methoxy-6-quinoli necarboxamide (Compound 11);
6-carbamoyl-4-(1-cyclopropylcarbamoyl-1H-indole-5-yloxy)-7-methoxyquinoli ne (Compound 12);
N6-(2-methoxyethyl)-4-(3-chloro-4-(((cyclopropylamino)carbonyl)amino)pheno xy)-7-methoxy-6-quinolinecarboxamide (Compound 13);
N1-phenyl-5-((2-(((1-methyl-4-piperidyl)carbonyl)amino)-4-pyridyl)oxy)-1H-1-indolecarboxamide (Compound 14);
N-(2-chloro-5-((6-cyano-7-(2-(1-pyrrolidino)ethoxy)-quinolyl)oxy)phenyl)-N'-c yclopropylurea (Compound 15);
N6-methyl-4-(4-chloro-3-(((methylamino)carbonyl)amino)phenoxy)-7-methoxy-6-quinolinecarboxamide (Compound 16);
N-(2-chloro-5-((6-cyano-7-((2R)-2-hydroxy-3-(1-pyrrolidino)propoxy)-4-quinol yloxy)phenyl)-N'-cyclopropylurea (Compound 17);
N6-methyl-4-(3-chloro-4-(((cyclopropylamino)carbonyl)amino)phenoxy)-7-(2-methoxyethoxy)-6-quinolinecarboxamide (Compound 18);
N6-methyl-4-(3-chloro-4-(((ethylamino)carbonyl)amino)phenoxy)-7-methoxy-6 -quinolinecarboxamide (Compound 19);
N6-ethyl-4-(3-chloro-4-(((ethylamino)carbonyl)amino)phenoxy)-7-methoxy-6-q uinolinecarboxamide (Compound 20);
N6-methyl-4-(3-chloro-4-(((ethylamino)carbonyl)amino)phenoxy)-7-((2R)-3-die thylamino-2-hydroxypropoxy-6-quinolinecarboxamide (Compound 21);
N6-methyl-4-(3-chloro-4-(((ethylamino)carbonyl)amino)phenoxy)-7-((2R)-2-hy droxy-3-(1-pyrrolidino)propoxy)-6-quinolinecarboxamide (Compound 22); and
N6-methyl-4-(3-chloro-4-(((ethylamino)carbonyl)amino)phenoxy)-7-((1-methyl-4-piperidyl)methoxy)-6-quinolinecarboxamide (Compound 23).

The compound represented by General Formula (II) can be produced by a known method, for example, by a method described in either International publication No. 02/32872 (WO02/32872) or International publication No. 2005/063713 (WO2005/063713).

According to the present invention, the compound represented by General Formula (I) may form a pharmacologically acceptable salt with acid or base. The compound of the invention also comprises such pharmacologically acceptable salts. Examples of salts formed with acids include inorganic acid salts such as hydrochloride salts, hydrobromate salts, sulfate salts and phosphate salts, and organic acid salts such as formic acid, acetic acid, lactic acid, succinic acid, fumaric acid, maleic acid, citric acid, tartaric acid, stearic acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, organic base salts such as trimethylamine, triethylamine, pyridine, picoline, dicyclohexylamine, N, N'-dibenzyl ethylenediamine, arginine and lysine and ammonium salts.

Furthermore, according to the present invention, solvates and enantiomers of the compound represented by General Formula (I) may exist. According to the present invention, the compound of the invention comprises such solvates and enantiomers. Examples of solvates include hydrates and nonhydrates, preferably hydrates. Examples of solvents include water, alcohols (for example, methanol, ethanol and n-propanol) and dimethylformamide.

Moreover, according to the present invention, the compound represented by General Formula (I) may be crystalline or amorphous. If a crystalline polymorph is present, it may be a single body or a mixture of any crystalline shape.

According to the present invention, the compound of the invention also comprises compounds that undergo metabolism such as oxidation, reduction and hydrolysis *in vivo.* According to the present invention, the compound of the invention also comprises compounds that generate the compound represented by General Formula (I) by undergoing metabolism such as oxidation, reduction and hydrolysis *in vivo.*

Preferably, the compound of the invention is a substance having activity of inhibiting DDR2 kinase activity (hereinafter, also referred to as "DDR2 kinase inhibitory activity") (DDR2 inhibitor). Herein, "DDR2 kinase activity" refers to activity of DDR2 to autophosphorylate or phosphorylate a tyrosine residue of other protein.

Examples of methods for determining DDR2 kinase inhibitory activity of the compound of the invention include cell free kinase assay, western blotting, cell growth assay and viability assay. Examples of the cell growth assay include tritium thymidine uptake method, MTT method, XTT method (cell counting kit-8 (Dojindo Laboratories)), AlamarBlue technique, Neutral Red technique, BrdU technique, Ki67 staining and PCNA staining. Examples of the viability assay include TUNNEL staining, Caspase-3 cleavage detection and PARP cleavage detection. These methods may be carried out according to conventional techniques (Blood. 2005, 105, 2941-2948., Molecular Cancer Therapeutics. 2005, 4, 787-798).

Hereinafter, an exemplary method for determining DDR2 kinase inhibitory activity will be described.

The DDR2 kinase inhibitory activity can be determined by cell free kinase assay.

DDR2 can be prepared by gene-engineering means according to a conventional method. For example, according to the method of Baculovirus Expression System, DDR2 may be expressed in an insect cell *(Spodoptera frugiperda* 9 (Sf9)) as human recombinant GST fusion protein, human recombinant histidine-tag fusion protein or the like. Furthermore, the expressed recombinant protein can be purified by affinity chromatography (e.g., GSH-agarose (Sigma) or Ni-NTA-agarose (Qiagen)). The purity and identification of the protein can be confirmed by SDS-PAGE, silver staining and western blotting using an antibody specific to DDR2.

The cell free kinase assay may be carried out as follows.

First, to each well of a plate (e.g., 96-well, 384-well, etc.), a mixture containing 20 µl of standard reaction solution, 5 µl of ATP solution, 5 µl of the test substance, 10 µl of solution containing 50 ng of DDR2 recombinant protein and 10 µl of solution containing 125 ng of biotinylated Poly(Glu, Tyr)_{4:1} can be added sequentially.

Alternatively, to each well of a plate (e.g., 96-well, 384-well, etc.), 20 ng of DDR2 recombinant protein, 35 µL of 25 mM Hepes solution (pH 7.4) containing 14 mM MgCl₂ and 5 µL of the test substance can be added, followed by further addition of 10 µL of ATP solution.

For example, the test substance used may be dissolved in a certain amount of dimethylsulfoxide and made into a 100-fold dilution with 1% BSA.

This kinase reaction solution (50 µl) may contain 60 mM HEPES-NaOH (pH 7.5), 3 mM MgCl₂, 3 mM MnCl₂, 3 µM Na-orthovanadate, 1.2 mM DTT, 50 µg/ml PEG₂₀₀₀₀, 1 µM ATP and the like. In this case, the ATP may be labeled with a radioactive isotope such as [γ-³²P]-ATP and [γ-³³P]-ATP. The ATP solution may be diluted with 25 mM Hepes solution.

The reaction solution may be incubated for a certain period of time, and then 50 µl of 2% (v/v) H₃PO₄ solution or 10 µL of 50 mM EDTA (pH 8.0) solution may be added to terminate the reaction.

Each well may be subjected to an appropriate washing procedure.

DDR2 kinase inhibitory activity can be assessed by determining the amount of ATP incorporation. When a radioactive isotope-labeled ATP mentioned above is used, the amount of ATP incorporation can be assessed by determination of the radioactivity captured on the plate with a scintillation counter.

Following this method, the DDR2 kinase inhibitory activity of the compound of the invention can be assessed.

### (3) Therapeutic agent and therapeutic method

The therapeutic agent of the invention comprises the compound represented by General Formula (I), a pharmacologically acceptable salt thereof or a solvate thereof and is an agent for treating hepatic fibrosis.

The therapeutic agent of the invention may be administered to a living organism, i.e., a mammal (e.g., human, rat, rabbit, sheep, pig, bovine, cat, dog, monkey, etc.).

The effect of treatment may be confirmed by observation of an x-ray picture, CT or the like, or by histopathological diagnosis of biopsy.

According to the present invention, a therapeutic agent for hepatic fibrosis also comprises a drug for improving hepatic fibrosis, a drug for preventing cirrhosis, a drug for preventing hepatic carcinoma and the like. Since the compound of the invention has suppressing activity against hepatic fibrillization, it is also useful as a hepatic fibrillization suppressor.

Where a therapeutic agent of the invention is used, the given dose of the compound of the invention differs depending on the degree of the symptom, age, sex, weight and sensitivity difference of the patient, administration mode, administration period, administration interval, nature, prescription and the type of the pharmaceutical formulation, and the type of the active element. Usually, but without limitation, the dose of the compound is 0.1-1000 mg/day, preferably 0.5-100 mg/day, more preferably 1-30 mg/day for an adult (weight 60 kg), which may be administered once to three times a day.

Although the therapeutic agent for hepatic fibrosis comprising the compound of the invention as an active ingredient may be used alone, it is usually mixed with appropriate additives and made into a formulation.

Examples of such additives include excipients, binders, lubricants, disintegrants, colorants, flavoring agents, emulsifiers, surfactants, solubilizing agents, suspending agents, tonicity agents, buffers, antiseptic agents, antioxidant agents, stabilizers, absorption promoters and the like that are generally used for medicine. If required, they may be used in combination. Examples of such additive are as follows.

Excipients: lactose, sucrose, glucose, cornstarch, mannitol, sorbitol, starch, alpha-starch, dextrin, crystalline cellulose, light anhydrous silicic acid, aluminum silicate, calcium silicate, magnesium aluminometasilicate and calcium hydrogen phosphate.

Binders: for example, polyvinyl alcohol, methyl cellulose, ethyl cellulose, gum arabic, tragacanth, gelatin, shellack, hydroxypropyl methylcellulose, hydroxypropylcellulose, carboxymethylcellulose sodium, polyvinylpyrrolidone and macrogol.

Lubricants: magnesium stearate, calcium stearate, sodium stearyl fumarate, talc, polyethyleneglycol and colloid silica.

Disintegrants: crystalline cellulose, agar, gelatin, calcium carbonate, sodium hydrogen carbonate, calcium citrate, dextrin, pectin, low substituted hydroxypropylcellulose, carboxymethylcellulose, carboxymethylcellulose calcium, croscarmellose sodium, carboxymethyl starch and carboxymethyl starch sodium.

Colorants: ferric oxide, yellow ferric oxide, carmine, caramel, beta-carotene, titanium oxide, talc, riboflavin sodium phosphate, yellow aluminum lake and the like that are approved as additives in drugs.

Flavoring agents: cocoa powder, menthol, aromatic acid, peppermint oil, camphor and cinnamon powder.

Emulsifiers or surfactants: stearyltriethanolamine, sodium lauryl sulfate, laurylaminopropionate, lecithin, glycerine monostearate, sucrose fatty acid ester and glycerine fatty acid ester.

Solubilizing agents: polyethyleneglycol, propylene glycol, benzyl benzoate, ethanol, cholesterol, triethanolamine, sodium carbonate, sodium citrate, Polysorbate 80 and nicotine acid amide.

Suspending agents: for example, in addition to the surfactants mentioned above, hydrophilic polymers such as polyvinyl alcohol, polyvinylpyrrolidone, methylcellulose, hydroxymethylcellulose, hydroxyethylcellulose and hydroxypropylcellulose.

Tonicity agents: glucose, sodium chloride, mannitol and sorbitol.

Buffers: buffers such as phosphate, acetate, carbonate, citrate and the like.

Antiseptic agents: methylparaben, propylparaben, chlorobutanol, benzyl alcohol, phenethyl alcohol, dehydroacetic acid and sorbic acid.

Antioxidant agents: hydrosulfate, ascorbic acid and alpha-tocopherol. Stabilizers: those generally used for medicine.

Absorption promoters: those generally used for medicine.

If required, components such as vitamins and amino acids may be blended.

Examples of the above-mentioned formulations include oral formulations such as tablets, dispersant, granule, fine granule, capsule, syrup, lozenge and inhaler; external formulations such as suppository, ointment, eye ointment, poultice strip, eye-drops, nasal drops, eardrops, skin patch and lotion; and injectable formulations.

The oral formulations mentioned above may be formulated by appropriately combining the additives mentioned above. If necessary, surface of these formulations may be coated.

The external formulations mentioned above may be formulated by appropriately combining the additives mentioned above, particularly excipients, binders, flavoring agents, emulsifiers, surfactants, solubilizing agents, suspending agent, tonicity agents, antiseptic agents, antioxidant agents, stabilizers and absorption promoters.

The injectable formulations mentioned above may be formulated by appropriately combining the additives mentioned above, particularly emulsifiers, surfactants, solubilizing agents, suspending agents, tonicity agents, buffers, antiseptic agents, antioxidant agents, stabilizers and absorption promoters. The injectable formulations may be used through means such as infusion, intramuscular injection, subcutaneous injection, intradermal injection and intravenous injection.

The present invention comprises a method for treating hepatic fibrosis, comprising administering an effective amount of a compound of the invention to a patient.

According to the method of the invention, the route and the method for administering the compound of the invention are not particularly limited and reference may be made to the description of the therapeutic agent of the invention for hepatic fibrosis.

The present invention relates to use of a compound of the invention for producing a therapeutic agent for hepatic fibrosis.

The present invention further relates to a compound of the invention for a therapeutic agent for hepatic fibrosis.

The present invention also provides a DDR2 inhibitor comprising a compound represented by General Formula (I), a pharmacologically acceptable salt thereof or a solvate thereof The DDR2 inhibitor of the invention is capable of inhibiting kinase activity of DDR2.

Although the compound represented by General Formula (I) is as described above, it is preferably 4-(3-chloro-4-(cyclopropylaminocarbonyl)aminophenoxy)-7-methoxy-6-quinolinecarbox amide.

DDR2 kinase inhibitory activity of a DDR2 inhibitor of the invention may be determined as described above.

As the DDR2 inhibitor of the invention, the compound of the invention may be used alone, or it may be mixed and formulated with appropriate additives mentioned above.

As to the usage and the dosage of the DDR2 inhibitor, reference may be made to the description of the therapeutic agent for hepatic fibrosis above.

The present invention also relates to use of a compound represented by General Formula (I), a pharmacologically acceptable salt thereof or a solvate thereof for producing a DDR2 inhibitor.

The present invention further relates to a compound represented by General Formula (I), a pharmacologically acceptable salt thereof or a solvate thereof for a DDR2 inhibitor.

The present invention yet further relates to a method for inhibiting kinase activity of DDR2 with a compound represented by General Formula (I), a pharmacologically acceptable salt thereof or a solvate thereof. According to the method of the invention, the usage and the dosage of the compound are not particularly limited and reference may be made to the description of the therapeutic agent for hepatic fibrosis above.

### EXAMPLES

Hereinafter, the present invention will be illustrated by way of specific examples, although the invention should not be limited thereto.

### [Example 1] Determination of DDR2 kinase inhibitory activity of compounds of the invention

### 1. Cloning of DDR2 and preparation of DDR2 recombinant Baculovirus solution

A DNA fragment of cytoplasmic domain of human DDR2 (SEQ ID NO: 1) was isolated by PCR method (Expand High Fidelity (purchased from Roche)) according to a conventional technique using human brain cDNA library (purchased from Clontech) as a template and SEQ ID NO:3: 5'-GAATTCAAAGATGTGGCTGTGGAGGAGTT-3' and SEQ ID NO:4: 5'-GCTCTAGAGCTCACTCGTCGCCTTGT-3' (purchased from JBioS) as primers. This DNA fragment was inserted into a baculovirus transplacement vector (pFastBac™-HT (purchased from Invitrogen)) to obtain a recombinant construct. Insect cells *(Spodoptera frugiperda 9* (Sf9 cells)) were transfected with this recombinant construct, thereby preparing a DDR2 recombinant baculovirus solution. For preparation of a recombinant baculovirus, we referred to a standard textbook (Bac-to-Bac Baculovirus Expression System (Invitrogen)).

### 2. Expression and purification of DDR2

The above-described DDR2 recombinant Baculovirus solution (0.07 mL) was added to Sf9 cells (7 x 10⁷ cells) suspended in 2.5% FBS-containing SF-900II medium (purchased from Invitrogen) and shake-cultured at 27°C for 72 hours. Then, this DDR2 recombinant Baculovirus-infected cells were subjected to centrifugation at 1,600 rpm at 4°C for 6 minutes and the supernatant was removed. The precipitated infected cells were suspended in 10 mL of ice-cold PBS and centrifuged at 1,600 rpm at 4°C for 6 minutes to remove the supernatant. The precipitated infected cells were suspended in 5 mL of ice-cold lysis buffer (50 mM Tris-HCl (pH 8.5), 5 mM 2-mercaptoethanol, 100 mM KCl, 1 mM PMSA, 1%(v/v) NP40), and centrifuged at 10,000 rpm at 4°C for 10 minutes to obtain the supernatant.

This supernatant was added to a Ni-NTA agarose column (1.5 mL (purchased from Qiagen)) equilibrated with 5 mL of buffer A (20 mM Tris-HCl (pH 8.5), 5 mM 2-mercaptoethanol, 500 mM KCl, 20 mM imidazole, 10% (v/v) glycerol). This column was sequentially washed with 10 mL of buffer A, 2 mL of buffer B (20 mM Tris-HCl (pH8.5), 5 mM 2-mercaptoethanol, 1 M KCl, 10% (v/v) glycerol) and 2 mL of buffer A. Subsequently, 4 mL of buffer C (20 mM Tris-HCl (pH 8.5), 5 mM 2-mercaptoethanol, 100 mM KCl, 100 mM imidazole, 10% (v/v) glycerol) was added to obtain an eluate. This eluate was placed in a dialysis membrane (purchased from Spectrum Medical Industries) and dialyzed against 1 L of dialysis buffer (20 mM Tris-HCl, 10% (v/v) glycerol, 1 mM dithiothreitol, 0.1 mM Na₃VO₄, 0.1 mM EGTA) at 4°C overnight. After dialysis, a portion of the eluate was subjected to SDS-PAGE and an amount of the recombinant protein detected at a molecular weight of about 40 kDa (His6-DDR2 (cytoplasmic domain of DDR2 having 6 histidines fused to the N-terminus thereof)) by Coomassie brilliant blue staining was quantitated using BSA as a standard substance.

### 3. Determination of DDR2 kinase inhibitory activity

To each well of a 96-well black half plate (purchased from Corning, Product No: 3694), 20 ng of His6-DDR2, 35 µL of 25 mM Hepes solution (pH 7.4) containing 14 mM MgCl₂ and a test substance dissolved in dimethylsulfoxide (at 100-fold dilution with 1% BSA; 5 µL) were added to a total amount of 40 µL. To this, 10 µL of 50 µM ATP (purchased with Sigma) diluted with 25 mM Hepes solution was added and incubated at room temperature for 10 minutes. Thereafter, 10 µL of 50 mM EDTA (pH 8.0) (purchased from Wako Pure Chemical Industries) was added to terminate the kinase reaction.

The test substances used were:
N-(4-methoxyphenyl)-N'-(4-((6,7-dimethoxy-4-quinolinyl)oxy)phenyl)urea (Compound 1);
N-n-butyl-N'-(4-((6,7-dimethoxy-4-quinolinyl)oxy)phenyl)urea (Compound 2);
N-(2-thiazole)-N'-(4-((6,7-dimethoxy-4-quinolinyl)oxy)phenyl)urea (Compound 3);
N-(3-carbamoylphenyl)-N'-(4-((6,7-dimethoxy-4-quinolinyl)oxy)phenyl)urea (Compound 4);
N-(3-methylthiophenyl)-N'-(4-((6,7-dimethoxy-4-quinolinyl)oxy)phenyl)urea (Compound 5);
N-(3-methylsulfonylphenyl)-N'-(4-((6,7-dimethoxy-4-quinolinyl)oxy)phenyl)ur ea (Compound 6);
N-(4-(6-cyano-7-(2-methoxyethoxy)-4-quinolyl)oxy-2-fluorophenyl)-N'-(3-met hylsulfonylphenyl)urea (Compound 7);
4-(4-((4-fluoroanilino)carbonyl)amino-3-fluorophenoxy)-7-methoxy-6-quinoline carboxamide (Compound 8);
6,7-dimethoxy-4-(5-(1-(4-fluorophenylcarbamoyl)-indolyl)oxy)quinoline (Compound 9);
4-(3-fluoro-4-((3-methylthiopropylamino)carbonyl)anunophenoxy)-7-methoxy-6-quinolinecarboxamide (Compound 10);
4-(3-chloro-4-(cyclopropylaminocarbonyl)aminophenoxy)-7-methoxy-6-quinoli necarboxamide (Compound 11);
6-carbamoyl-4-(1-cyclopropylcarbamoyl-1H-indole-5-yloxy)-7-methoxyquinoli ne (Compound 12);
N6-(2-methoxyethyl)-4-(3-chloro-4-(((cyclopropylamino)carbonyl)amino)pheno xy)-7-methoxy-6-quinolinecarboxamide (Compound 13);
N1-phenyl-5-((2-(((1-methyl-4-piperidyl)carbonyl)amino)-4-pyridyl)oxy)-1H-1-indolecarboxamide (Compound 14);
N-(2-chloro-5-((6-cyano-7-(2-(1-pyrrolidino)ethoxy)-quinolyl)oxy)phenyl)-N'-c yclopropylurea (Compound 15);
N6-methyl-4-(4-chloro-3-(((methylamino)carbonyl)amino)phenoxy)-7-methoxy-6-quinolinecarboxamide (Compound 16);
N-(2-chloro-5-((6-cyano-7-((2R)-2-hydroxy-3-(1-pyrrolidino)propoxy)-4-quinol yloxy)phenyl)-N'-cyclopropylurea (Compound 17);
N6-methyl-4-(3-chloro-4-(((cyclopropylamino)carbonyl)amino)phenoxy)-7-(2-methoxyethoxy)-6-quinolinecarboxamide (Compound 18);
N6-methyl-4-(3-chloro-4-(((ethylamino)carbonyl)amino)phenoxy)-7-methoxy-6 -quinolinecarboxamide (Compound 19);
N6-ethyl-4-(3-chloro-4-(((ethylamino)carbonyl)amino)phenoxy)-7-methoxy-6-q uinolinecarboxamide (Compound 20);
N6-methyl-4-(3-chloro-4-(((ethylamino)carbonyl)amino)phenoxy)-7-((2R)-3-die thylamino-2-hydroxypropoxy-6-quinolinecarboxamide (Compound 21);
N6-methyl-4-(3-chloro-4-(((ethylamino)carbonyl)amino)phenoxy)-7-((2R)-2-hy droxy-3-(1-pyrrolidino)propoxy)-6-quinolinecarboxamide (Compound 22); and
N6-methyl-4-(3-chloro-4-(((ethylamino)carbonyl)amino)phenoxy)-7-((1-methyl-4-piperidyl)methoxy)-6-quinolinecarboxamide (Compound 23).

The test substances were produced according to the description of International Publication No. 02/32872 (WO02/32872).

Tyrosine-phosphorylated DDR2 was detected using homogeneous time resolved fluorescence (HTRF) technique (Analytical Biochemistry, 269, 94-104, 1999). Specifically, to each well of the reaction solutions mentioned above, 20 µL of 25 mM Hepes solution containing 1 M KF, 4.75 ng of europium cryptate-labeled anti-phosphorylated tyrosine antibody (Eu(K)-PT66) and 100 ng of XL665-labeled anti-His antibody (XL665-Anti-His) was added. This reaction solution was incubated at room temperature overnight. Thereafter, fluorescence intensities of each well at 665 nm and 620 nm were measured under irradiation at an excitation wavelength of 337 nm using Discovery HTRF microplate analyzer (from Packard). The tyrosine autophosphorylation rate of His6-DDR2 was calculated using deltaF% described in HTRF standard experiment textbook from Nihon Schering. Specifically, a rate (%) of deltaF% of each well added with the test substance was determined where deltaF% of the well without the addition of the test substance and only with His6-DDR2 was assumed 100% while deltaF% of the well without the addition of both the test substance and His6-DDR2 was assumed 0%. Based on these rates (%), concentration of the test substance required for inhibiting His6-DDR2 kinase activity by 50% (IC₅₀) was calculated (Table 1)

**Table 1**

| Compound No. | IC₅₀ (nM) | Compound No. | IC₅₀ (nM) |
|---|---|---|---|
| 1 | 0.06 | 13 | 18 |
| 2 | 7.2 | 14 | 0.24 |
| 3 | 0.18 | 15 | 822 |
| 4 | 1.3 | 16 | 1890 |
| 5 | 1.2 | 17 | 20.8 |
| 6 | 1.1 | 18 | 0.4 |
| 7 | 0.63 | 19 | 178 |
| 8 | 1.2 | 20 | 428 |
| 9 | 1.9 | 21 | 28 |
| 10 | 1.5 | 22 | 0.4 |
| 11 | 0.91 | 23 | 18 |
| 12 | 0.15 | | |

Table 1 shows DDR2 kinase inhibitory activity (IC₅₀) of each compound.

As a result, all compounds were found to have DDR2 kinase inhibitory activity.

### [Example 2] Effect of compound of the invention in mouse models of thioacetamide-induced cirrhosis

Thioacetamide (purchased from Tokyo Chemical Industry) was intraperitoneally administered at 150 mg/kg for the first week and 200 mg/kg for the next second to fifth weeks to Balb/cAnN female mice (purchased from Charles River Laboratories) twice a week. The test substance (Compound 3) was suspended in 0.5% methylcellulose aqueous solution and orally administered once daily at 1 or 3 mg/kg. Three days after the final thioacetamide administration, livers were removed from the mice and subjected to 10% neutral buffered formalin fixation followed by Azan staining (staining technique is described in "Medical Technology, supp., new technique for staining" (Ishiyaku Publisher, 1999)). The degree of fibrillization was examined by observation of the stained tissue sections. Experiments were conducted for a group without the administration of thioacetamide (6 examples), a group administered with thioacetamide but a test substance (3 examples), a group administered with Compound 3 at 1 mg/kg (6 examples) and a group administered with Compound 3 at 3 mg/kg (3 examples).

As a result, Compound 3 suppressed hepatic fibrillization in a dose-dependent manner. Representative result of staining for each group is shown in Figure 1.

These results show that the compound of the invention has activity of suppressing hepatic fibrillization. The compound of the invention was also shown to be useful as a therapeutic agent for hepatic fibrosis.

### [Reference Example]

Hereinafter, a method for producing a formulation of one of the compounds of the invention, i.e., 4-(3-chloro-4-(cyclopropylaminocarbonyl)aminophenoxy)-7-methoxy-6-quinolinecarbox amide, will be described as a reference example.

### (Production of pharmaceutical composition)

### (1) 1 mg tablet

24g of crystal (C) of methanesulfonate of 4-(3-chloro-4-(cyclopropylaminocarbonyl)aminophenoxy)-7-methoxy-6-quinolinecarbox amide (hereinafter, also referred to as "crystal (C)", which was produced according to the method described in Example 7 of W02005/063713) and 192g of light anhydrous silicic acid (antigelling agent sold under the trade name of AEROSIL(registered trademark) 200, Nippon Aerosil) were mixed with 20L Super Mixer, and then 1236g ofD-mannitol (excipient, Towa-Kasei), 720g of crystalline cellulose (excipient sold under the trade name of Avicel PH101, Asahi Kasei) and 72g of hydroxypropylcellulose (binder sold under the trade name of HPC-L, Nippon Soda) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 120g of croscarmellose sodium (disintegrant sold under the trade name of Ac-Di-Sol, FMC International Inc.) and 36g of sodium stearyl fumarate (lubricant, JRS Pharma LP) were placed and mixed together in a 20L tumbler mixer, and molded with a tablet machine to obtain tablets with a total mass of 100 mg per tablet. Furthermore, the tablets were coated using aqueous 10% Opadry yellow (OPADRY 03F42069 YELLOW, Colorcon Japan) solution as a coating solution with a tablet coating machine, thereby obtaining coated tablets with a total mass of 105 mg per tablet.

### (2) 10 mg tablet

60g of crystal (C) and 192g of light anhydrous silicic acid (antigelling agent sold under the trade name of AEROSIL(registered trademark) 200, Nippon Aerosil) were mixed with 20L Super Mixer, and then 1200g of D-mannitol (excipient, Towa-Kasei), 720g of crystalline cellulose (excipient sold under the trade name of Avicel PH101, Asahi Kasei) and 72g of hydroxypropylcellulose (binder sold under the trade name of HPCL, Nippon Soda) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 120g of croscarmellose sodium (disintegrant sold under the trade name of Ac-Di-Sol, FMC International Inc.) and 36g of sodium stearyl fumarate (lubricant, JRS Pharma LP) were placed and mixed together in a 20L tumbler mixer, and molded with a tablet machine to obtain tablets with a total mass of 400 mg per tablet. Furthermore, the tablets were coated using aqueous 10% Opadry yellow (OPADRY 03F42069 YELLOW, Colorcon Japan) solution as a coating solution with a tablet coating machine, thereby obtaining coated tablets with a total mass of 411 mg per tablet.

### (3) 100 mg tablet

31.4g of crystal (C) and 4g of light anhydrous silicic acid (antigelling agent sold under the trade name of AEROSIL(registered trademark) 200, Nippon Aerosil) were mixed with 1L Super Mixer, and then 40.1g of anhydrous calcium hydrogen phosphate (excipient, Kyowa Chemical Industry), 10g of low substituted hydroxypropylcellulose (binder sold under the trade name ofL-HPC (LH-21), Shin-Etsu Chemical) and 3g of hydroxypropylcellulose (binder sold under the trade name of HPC-L, Nippon Soda) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 10g of croscarmellose sodium (disintegrant sold under the trade name of Ac-Di-Sol, FMC International Inc.) and 1.5g of sodium stearyl fumarate (lubricant, JRS Pharma LP) were mixed and molded with a tablet machine to obtain tablets with a total mass of 400 mg per tablet.

### INDUSTRIAL APPLICABILITY

According to the present invention, there is provided a therapeutic agent for hepatic fibrosis comprising a compound represented by General Formula (I), a pharmacologically acceptable salt thereof or a solvate thereof, a method for treating hepatic fibrosis, use of the compound represented by General Formula (I), a pharmacologically acceptable salt thereof or a solvate thereof for producing the therapeutic agent for hepatic fibrosis, and the compound represented by General Formula (I), a pharmacologically acceptable salt thereof or a solvate thereof for the therapeutic agent for hepatic fibrosis.

Furthermore, the present invention also provides a DDR2 inhibitor comprising the compound represented by General Formula (I), a pharmacologically acceptable salt thereof or a solvate thereof.

### SEQUENCE LISTING Free Text

SEQ ID NO: 3 Primer
SEQ ID NO: 4 Primer

## Claims

1. A therapeutic agent for hepatic fibrosis comprising a compound represented by the following General Formula (I), a pharmacologically acceptable salt thereof, or a solvate of said compound or said salt, 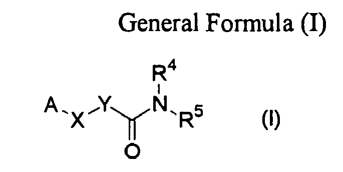 [wherein, A represents a group represented by any one of the following formulae 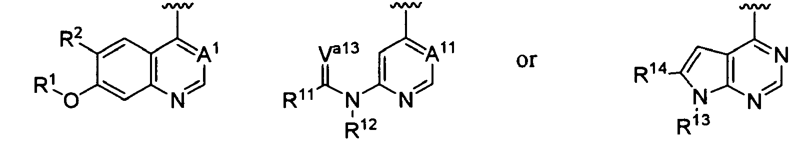
(wherein, R¹ represents a group represented by Formula V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
A¹ represents an optionally substituted carbon atom or nitrogen atom;
R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group or an optionally substituted mono-C₁₋₆ alkylamino group; R¹² represents a hydrogen atom or an optionally substituted C₁₋₆ alkyl group;
V^{a13} represents an oxygen atom or a sulfur atom;
A¹¹ represents an optionally substituted carbon atom or nitrogen atom;
R¹³ represents a hydrogen atom, an optionally substituted C₁₋₆ alkyl group or an optionally substituted C₃₋₈ cycloalkyl group;
R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group));
X represents an oxygen atom or a sulfur atom;
Y represents a group represented by any one of the following formulae 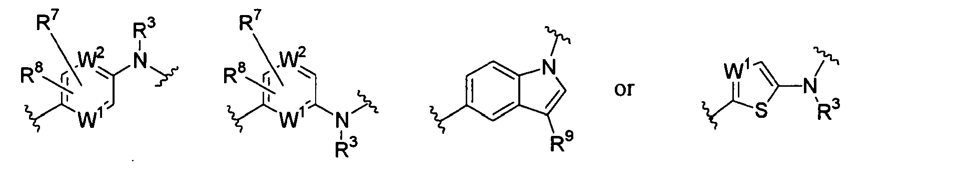
(wherein, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
R⁷ and k⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group);
R⁹ represents a hydrogen atom, a halogen atom or an optionally substituted C₁₋₆ alkyl group;
W¹ and W² each independently represent an optionally substituted carbon atom or nitrogen atom);
R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group; and
R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group].

2. The therapeutic agent according to Claim 1, wherein the compound represented by General Formula (I), a pharmacologically acceptable salt thereof, or a solvate of said compound or said salt is a compound represented by the following General Formula (II), a pharmacologically acceptable salt thereof, or a solvate of said compound or said salt, 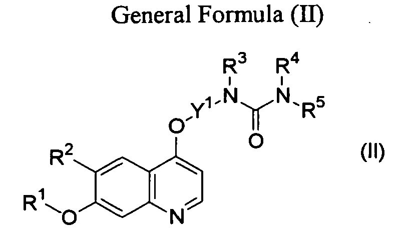
[wherein, R¹ represents a group represented by Formula V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
Y¹ represents a group represented by the following formula 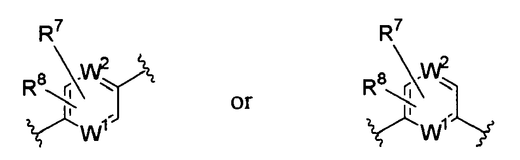
(wherein, R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{a1}V^{d2} (wherein, V^{a1} and V^{a2} each independently represent a hydrogen atom or an optionally substituted C₁₋₆ alkyl group; and W¹ and W² each independently represent an optionally substituted carbon atom or nitrogen atom);
R³ and R⁴ each independently represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group; and
R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group].

3. The therapeutic agent according to Claim 2, wherein R¹ is a C₁₋₆ alkyl group (provided that R¹ may have at least one substituent selected from the group consisting of a 3-10-membered nonaromatic heterocyclic group which may have a C₁₋₆ alkyl group, a hydroxyl group, a C₁₋₆ alkoxy group, an amino group, a mono-C₁₋₆ alkylamino group and a di-C₁₋₆ alkylamino group).

4. The therapeutic agent according to Claim 2, wherein R¹ is a methyl group or a group represented by any one of the following formulae 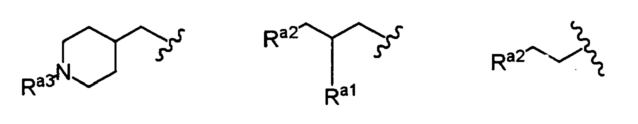 (wherein, R^{a3} represents a methyl group; R^{a1} represents a hydrogen atom or a hydroxyl group; R^{a2} represents a methoxy group, an methoxy group, a 1-pyrrolidinyl group, a 1-piperidinyl group, a 4-morpholinyl group, a dimethylamino group or a diethylamino group).

5. The therapeutic agent according to Claim 2, wherein R¹ is a methyl group or a 2-methoxyethyl group.

6. The therapeutic agent according to Claim 2, wherein R² is a cyano group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group).

7. The therapeutic agent according to Claim 2, wherein R² is a cyano group or a group represented by Formula -CONHV^{a16} (wherein, V^{a16} represents a hydrogen atom, a C₁₋₆ alkyl group, a C₃₋₈ cycloalkyl group, a C₁₋₆ alkoxy group or a C₃₋₈ cycloalkoxy group, provided that V^{a16} may have at least one substituent selected from the group consisting of a halogen atom, a cyano group, a hydroxyl group and a C₁₋₆ alkoxy group).

8. The therapeutic agent according to Claim 2, wherein R² is a group represented by Formula -CONHV^{a17} (wherein, V^{a17} represents a hydrogen atom, a C₁₋₆ alkyl group or a C₁₋₆ alkoxy group).

9. The therapeutic agent according to Claim 2, wherein R² is a group represented by Formula -CONHV^{a18} (wherein, V^{a18} represents a hydrogen atom, a methyl group or a methoxy group)

10. The therapeutic agent according to Claim 2, wherein Y¹ is a group represented by the following formula 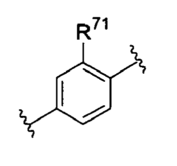 (wherein, R⁷¹ represents a hydrogen atom or a halogen atom).

11. The therapeutic agent according to Claim 2, wherein R³ and R⁴ are hydrogen atoms.

12. The therapeutic agent according to Claim 2, wherein R⁵ is a hydrogen atom, a C₁₋₆ alkyl group, a C₃₋₈ cycloalkyl group or a C₆₋₁₀ aryl group (provided that R⁵ may have at least one substituent selected from the group consisting of a halogen atom and a methanesulfonyl group).

13. The therapeutic agent according to Claim 2, wherein R⁵ is a methyl group, an ethyl group or a cyclopropyl group.

14. The therapeutic agent according to Claim 1, wherein the compound represented by General Formula (I), a pharmacologically acceptable salt thereof, or a solvate of said compound or said salt is at least one compound selected from the group consisting of:
N-(4-(6-cyano-7-(2-methoxyethoxy)-4-quinolyl)oxy-2-fluorophenyl)-N'-(4-fluor ophenyl)urea;
N-(2-chloro-4-((6-cyano-7-((1-methyl-4-piperidyl)methoxy)-4-quinolyl)oxy)phe nyl)-N'-cyclopropylurea;
N-(4-((6-cyano-7-(((2R)-3-(diethylamino)-2-hydroxypropyl)oxy)-4-quinolyl)oxy )phenyl)-N'-(4-fluorophenyl)urea;
N-(4-((6-cyano-7-(((2R)-2-hydroxy-3-(1-pyrrolidino)propyl)oxy)-4-quinolyl)ox y)phenyl)-N'-(4-fluorophenyl)urea;
4-(3-chloro-4-(cyclopropylaminocarbonyl)aminophenoxy)-7-methoxy-6-quinoli necarboxamide;
4-(3-chloro-4-(cyclopropylaminocarbonyl)aminophenoxy)-7-(2-methoxyethoxy) -6-quinolinecarboxamide;
N6-cyclopropyl-4-(3-chloro-4-(((cyclopropylamino)carbonyl)amino)phenoxy)-7 -methoxy-6-quinolinecarboxamide;
N6-(2-methoxyethyl)-4-(3-chloro-4-(((cyclopropylamino)carbonyl)amino)pheno xy)-7-methoxy-6-quinolinecarboxamide;
N6-(2-fluoroethyl)-4-(3-chloro-4-(((cyclopropylamino)carbonyl)amino)phenoxy )-7-methoxy-6-quinolinecarboxamide;
N6-methoxy-4-(3-chloro-4-(((cyclopropylamino)carbonyl)amino)phenoxy)-7-m ethoxy-6-quinolinecarboxamide;
N6-methyl-4-(3-chloro-4-(((cyclopropylamino)carbonyl)amino)phenoxy)-7-met hoxy-6-quinolinecarboxamide;
N6-ethyl-4-(3-chloro-4-(((cyclopropylamino)carbonyl)amino)phenoxy)-7-metho xy-6-quinolinecarboxamide;
4-(3-fluoro-4-(cyclopropylaminocarbonyl)aminophenoxy)-7-(2-methoxyethoxy) -6-quinolinecarboxamide;
4-(3-chloro-4-(cyclopropylaminocarbonyl)aminophenoxy)-7-(2-hydroxyethoxy)-6-quinolinecarboxamide;
4-(3-chloro-4-(cyclopropylaminocarbonyl)aminophenoxy)-7-((2S)-2,3-dihydrox ypropyl)oxy-6-quinolinecarboxamide;
4-(3-chloro-4-(methylaminocarbonyl)aminophenoxy)-7-methoxy-6-quinolinecar boxamide;
4-(3-chloro-4-(ethylaminocarbonyl)aminophenoxy)-7-methoxy-6-quinolinecarbo xamide;
N6-methoxy-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-ethoxyethoxy)-6 -quinolinecarboxamide;
4-(4-((cyclopropylamino)carbonyl)aminophenoxy)-7-(2-methoxyethoxy)-6-quin olinecarboxamide;
N-(2-fluoro-4-((6-carbamoyl-7-methoxy-4-quinolyl)oxy)phenyl)-N'-cyclopropyl urea;
N6-(2-hydroxyethyl)-4-(3-chloro-4-(((cyclopropylamino)carbonyl)amino)pheno xy)-7-methoxy-6-quinolinecarboxamide;
4-(3-chloro-4-(1-propylaminocarbonyl)aminophenoxy)-7-methoxy-6-quinolinec arboxamide;
4-(3-chloro-4-(cis-2-fluoro-cyclopropylaminocarbonyl)aminophenoxy)-7-metho xy-6-quinolinecarboxamide;
N6-methyl-4-(3-chloro-4-(((cyclopropylamino)carbonyl)amino)phenoxy)-7-(2-methoxyethoxy)-6-quinolinecarboxamide;
N6-methyl-4-(3-chloro-4-(((ethylamino)carbonyl)amino)phenoxy)-7-methoxy-6-quinolinecarboxamide;
4-(3-chloro-4-(cyclopropylaminocarbonyl)aminophenoxy)-7-(2-(4-morpholino)e thoxy)-6-quinolinecarboxamide;
4-(3-chloro-4-(2-fluoroethylaminocarbonyl)aminophenoxy)-7-methoxy-6-quinol inecarboxamide;
N6-((2R)tetrahydro-2-furanylmethyl)-4-(3-chloro-4-(((methylamino)carbonyl)a mino)phenoxy)-7-methoxy-6-quinolinecarboxamide;
4-(3-fluoro-4-(ethylaminocarbonyl)aminophenoxy)-7-methoxy-6-quinolinecarbo xamide;
4-(3-chloro-4-(((cyclopropylamino)carbonyl)amino)phenoxy)-7-((2R)-2-hydrox y-3-(1-pyrrolidino)propoxy)-6-quinolinecarboxamide;
N6-methyl-4-(3-chloro-4-(((methylamino)carbonyl)amino)phenoxy)-7-((2R)-3-d iethylamino-2-hydroxypropoxy)-6-quinolinecarboxamide;
N6-methyl-4-(3-chloro-4-(((ethylamino)carbonyl)amino)phenoxy)-7-((2R)-3-die thylamino-2-hydroxypropoxy)-6-quinolinecarboxamide;
N6-methyl-4-(3-chloro-4-(((methylamino)carbonyl)amino)phenoxy)-7-((2R)-2-h ydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hy droxy-3-(1-pyrrolidino)propoxy)-6-quinolinecarboxamide;
N6-methyl-4-(3-chloro-4-(((methylamino)carbonyl)amino)phenoxy)-7-((1-meth yl-4-piperidyl)methoxy)-6-quinolinecarboxamide;
N6-methyl-4-(3-chloro-4-(((ethylamino)carbonyl)amino)phenoxy)-7-((1-methyl-4-piperidyl)methoxy)-6-quinolinecarboxamide;
N-(4-(6-cyano-7-(2-methoxyethoxy)-4-quinolyl)oxy-2-fluorophenyl)-N'-cyclopr opylurea;
N-(4-(6-cyano-7-(3-(4-morpholino)propoxy)-4-quinolyl)oxyphenyl)-N'-(3-(meth ylsulfonyl)phenyl)urea;
4-(4-((cyclopropylamino)carbonyl)aminophenoxy)-7-methoxy-6-quinolinecarbo xamide;
4-(3-fluoro-4-((2-fluoroethylamino)carbonyl)aminophenoxy)-7-methoxy-6-quin olinecarboxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N '-cyclopropylurea,
a pharmacologically acceptable salt thereof, or a solvate of said compound or said salt.

15. The therapeutic agent according to Claim 1, wherein the compound represented by General Formula (I), a pharmacologically acceptable salt thereof, or a solvate of said compound or said salt is at least one compound selected from the group consisting of:
4-(3-chloro-4-(cyclopropylaminocarbonyl)aminophenoxy)-7-methoxy-6-quinoli necarboxamide;
4-(3-chloro-4-(ethylaminocarbonyl)aminophenoxy)-7-methoxy-6-quinolinecarbo xamide;
N6-methoxy-4-(3-chloro-4-(((cyclopropylamino)carbonyl)amino)phenoxy)-7-m ethoxy-6-quinolinecarboxamide;
4-(3-chloro-4-(methylaminocarbonyl)aminophenoxy)-7-methoxy-6-quinolinecar boxamide; and
N6-methoxy-4-(3-chloro-4-(((ethylamino)carbonyl)amino)phenoxy)-7-methoxy-6-quinolinecarboxamide,
a pharmacologically acceptable salt thereof, or a solvate of said compound or said salt.

16. The therapeutic agent according to Claim 1, wherein the compound represented by General Formula (I), a pharmacologically acceptable salt thereof, or a solvate of said compound or said salt is 4-(3-chloro-4-(cyclopropylaminocarbonyl)aminophenoxy)-7-methoxy-6-quinolinecarbox amide, a pharmacologically acceptable salt thereof, or a solvate of said compound or said salt.

17. The therapeutic agent according to Claim 1, wherein the compound represented by General Formula (I), a pharmacologically acceptable salt thereof, or a solvate of said compound or said salt is methanesulfonate of 4-(3-chloro-4-(cyclopropylaminocarbonyl)aminophenoxy)-7-methoxy-6-quinolinecarbox amide.

18. The therapeutic agent according to Claim 1, wherein the compound represented by General Formula (I), a pharmacologically acceptable salt thereof, or a solvate of said compound or said salt has DDR2 kinase inhibitory activity.

19. A method for treating hepatic fibrosis comprising administering an effective amount of a compound represented by the following General Formula (I), a pharmacologically acceptable salt thereof, or a solvate of said compound or said salt to a patient, 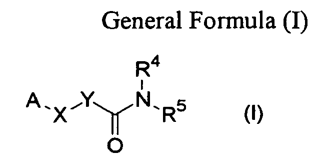 [wherein, A represents a group represented by any one of the following formulae 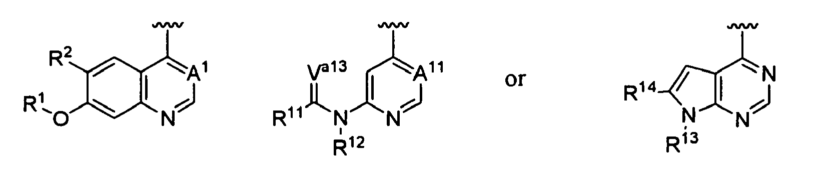
(wherein, R¹ represents a group represented by Formula -V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
A¹ represents an optionally substituted carbon atom or nitrogen atom;
R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group or an optionally substituted mono-C₁₋₆ alkylamino group; R¹² represents a hydrogen atom or an optionally substituted C₁₋₆ alkyl group;
V^{a13} represents an oxygen atom or a sulfur atom;
A¹¹ represents an optionally substituted carbon atom or nitrogen atom;
R¹³ represents a hydrogen atom, an optionally substituted C₁₋₆ alkyl group or an optionally substituted C₃₋₈ cycloalkyl group;
R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group));
X represents an oxygen atom or a sulfur atom;
Y represents a group represented by any one of the following formulae 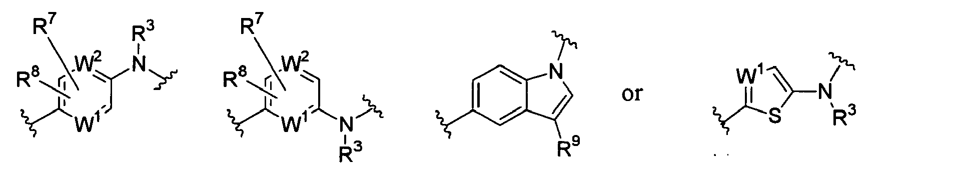
(wherein, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group);
R⁹ represents a hydrogen atom, a halogen atom or an optionally substituted C₁₋₆ alkyl group;
W¹ and W² each independently represent an optionally substituted carbon atom or nitrogen atom);
R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group; and
R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group].

20. Use of a compound represented by the following General Formula (I), a pharmacologically acceptable salt thereof, or a solvate of said compound or said salt for producing a therapeutic agent for hepatic fibrosis, 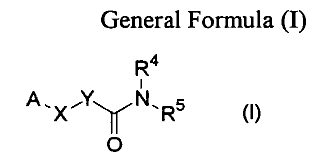 [wherein, A represents a group represented by any one of the following formulae 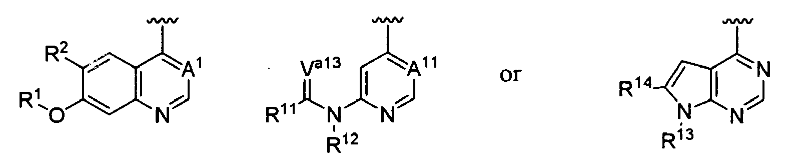
(wherein, R¹ represents a group represented by Formula -V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
A¹ represents an optionally substituted carbon atom or nitrogen atom;
R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group or an optionally substituted mono-C₁₋₆ alkylamino group; R¹² represents a hydrogen atom or an optionally substituted C₁₋₆ alkyl group;
V^{a13} represents an oxygen atom or a sulfur atom;
A¹¹ represents an optionally substituted carbon atom or nitrogen atom;
R¹³ represents a hydrogen atom, an optionally substituted C₁₋₆ alkyl group or an optionally substituted C₃₋₈ cycloalkyl group;
R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group));
X represents an oxygen atom or a sulfur atom;
Y represents a group represented by any one of the following formulae 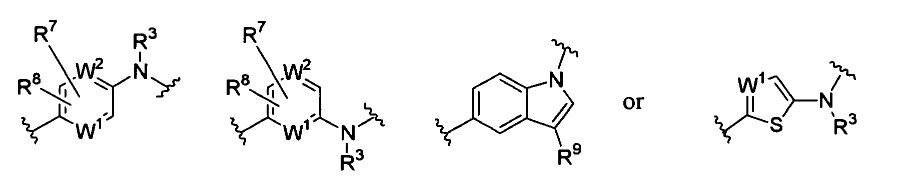
(wherein, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group);
R⁹ represents a hydrogen atom, a halogen atom or an optionally substituted C₁₋₆ alkyl group;
W¹ and W² each independently represent an optionally substituted carbon atom or nitrogen atom);
R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group; and
R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group].

21. The compound represented by the following General Formula (I), a pharmacologically acceptable salt thereof, or a solvate of said compound or said salt for a therapeutic agent for hepatic fibrosis, 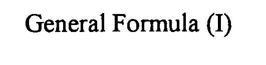 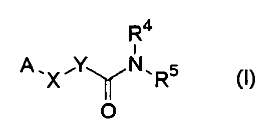 [wherein, A represents a group represented by any one of the following formulae 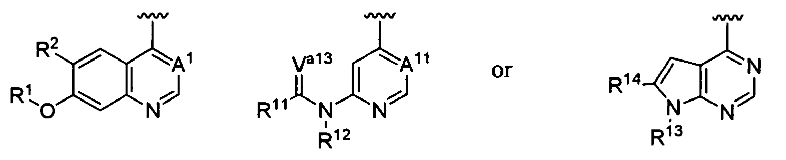
(wherein, R¹ represents a group represented by Formula -V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
A¹ represents an optionally substituted carbon atom or nitrogen atom;
R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group or an optionally substituted mono-C₁₋₆ alkylamino group; R¹² represents a hydrogen atom or an optionally substituted C₁₋₆ alkyl group;
V^{a13} represents an oxygen atom or a sulfur atom;
A¹¹ represents an optionally substituted carbon atom or nitrogen atom;
R¹³ represents a hydrogen atom, an optionally substituted C₁₋₆ alkyl group or an optionally substituted C₃₋₈ cycloalkyl group;
R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group));
X represents an oxygen atom or a sulfur atom;
Y represents a group represented by any one of the following formulae 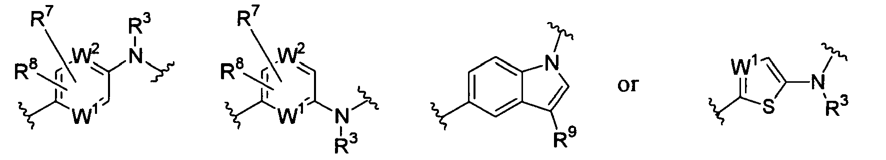
(wherein, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂-₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group);
R⁹ represents a hydrogen atom, a halogen atom or an optionally substituted C₁₋₆ alkyl group;
W¹ and W² each independently represent an optionally substituted carbon atom or nitrogen atom);
R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group; and
R⁵ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group].

22. A DDR2 inhibitor comprising a compound represented by 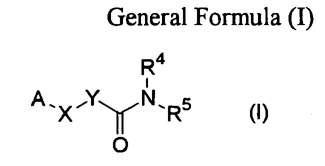 [wherein, A represents a group represented by any one of the following formulae 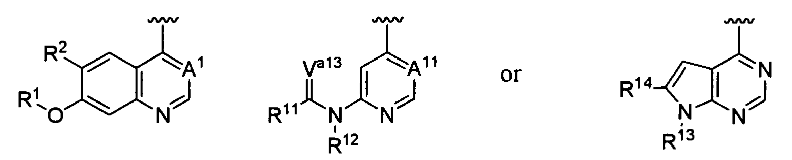
(wherein, R¹ represents a group represented by Formula -V¹-V²-V³ (wherein, V¹ represents an optionally substituted C₁₋₆ alkylene group; V² represents a single bond, an oxygen atom, a sulfur atom, a carbonyl group, a sulfinyl group, a sulfonyl group, a group represented by Formula -CONR⁶-, a group represented by Formula -SO₂NR⁶-, a group represented by Formula -NR⁶SO₂-, a group represented by Formula -NR⁶CO- or a group represented by Formula -NR⁶- (wherein, R⁶ represents a hydrogen atom, an optionally substituted C₁₋₆ alkyl group or an optionally substituted C₃₋₈ cycloalkyl group); V³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group);
R² represents a cyano group, an optionally substituted C₁₋₆ alkoxy group, a carboxyl group, an optionally substituted C₂₋₇ alkoxycarbonyl group or a group represented by Formula -CONV^{a11}V^{a12} (wherein, V^{a11} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group; V^{a12} represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, a hydroxyl group, an optionally substituted C₁₋₆ alkoxy group or an optionally substituted C₃₋₈ cycloalkoxy group);
A¹ represents an optionally substituted carbon atom or nitrogen atom;
R¹¹ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group or an optionally substituted mono-C₁₋₆ alkylamino group; R¹² represents a hydrogen atom or an optionally substituted C₁₋₆ alkyl group;
V^{a13} represents an oxygen atom or a sulfur atom;
A¹¹ represents an optionally substituted carbon atom or nitrogen atom;
R¹³ represents a hydrogen atom, an optionally substituted C₁₋₆ alkyl group or an optionally substituted C₃₋₈ cycloalkyl group;
R¹⁴ represents a group represented by Formula -V^{a14}-V^{a15} (wherein, V^{a14} represents a single bond or a carbonyl group; V^{a15} represents a hydrogen atom, a hydroxyl group, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group, an optionally substituted 3-10-membered nonaromatic heterocyclic group, an amino group, an optionally substituted mono-C₁₋₆ alkylamino group, an optionally substituted di-C₁₋₆ alkylamino group, a formyl group, a carboxyl group or an optionally substituted C₂₋₇ alkoxycarbonyl group));
X represents an oxygen atom or a sulfur atom;
Y represents a group represented by any one of the following formulae 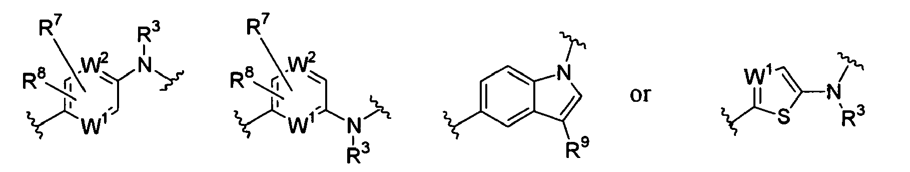
(wherein, R³ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group;
R⁷ and R⁸ each independently represent a hydrogen atom, a halogen atom, a cyano group, a nitro group, an amino group, an optionally substituted C₁₋₆ alkyl group, an optionally substituted C₃₋₈ cycloalkyl group, an optionally substituted C₁₋₆ alkoxy group, an optionally substituted C₁₋₆ alkylthio group, a formyl group, an optionally substituted C₂₋₇ acyl group, an optionally substituted C₂₋₇ alkoxycarbonyl group or a group represented by Formula -CONV^{d1}V^{d2} (wherein, V^{d1} and V^{d2} each independently represent a hydrogen atom or an optionally substituted C₁₋₆ alkyl group);
R⁹ represents a hydrogen atom, a halogen atom or an optionally substituted C₁₋₆ alkyl group;
W¹ and W² each independently represent an optionally substituted carbon atom or nitrogen atom);
R⁴ represents a hydrogen atom, an optionally substituted C₁₋₆ alkyl group, an optionally substituted C₂₋₆ alkenyl group, an optionally substituted C₂₋₆ alkynyl group, an optionally substituted C₃₋₈ cycloalkyl group, an optionally substituted C₂₋₇ acyl group or an optionally substituted C₂₋₇ alkoxycarbonyl group; and
R⁵ represents a hydrogen atom, an optionally substituted C₁₋₆ alkyl group, an optionally
substituted C₂₋₆ alkenyl group, an optionally substituted C₂₋₆ alkynyl group, an optionally substituted C₃₋₈ cycloalkyl group, an optionally substituted C₆₋₁₀ aryl group, an optionally substituted 5-10-membered heteroaryl group or an optionally substituted 3-10-membered nonaromatic heterocyclic group],
a pharmacologically acceptable salt thereof or a solvate thereof.


SEQUENCE LISTING

<110>  Eisai R&D Management Co., Ltd.

<120>  Therapeutic agent for liver fibrosis

<130>  G08-0072

<140>  PCT/JP2007/063525
<141>  2007-06-29

<150>  US60/817,872
<151>  2006-06-29

<160>  4

<170>  PatentIn version 3.3

<210>  1
<211>  2568
<212>  DNA
<213>  Homo sapiens

<400>  1
atgatcctga ttcccagaat gctcttggtg ctgttcctgc tgctgcctat cttgagttct      60

gcaaaagctc aggttaatcc agctatatgc cgctatcctc tgggcatgtc aggaggccag     120

attccagatg aggacatcac agcttccagt cagtggtcag agtccacagc tgccaaatat     180

ggaaggctgg actcagaaga aggggatgga gcctggtgcc ctgagattcc agtggaacct     240

gatgacctga aggagtttct gcagattgac ttgcacaccc tccattttat cactctggtg     300

gggacccagg ggcgccatgc aggaggtcat ggcatcgagt ttgcccccat gtacaagatc     360

aattacagtc gggatggcac tcgctggatc tcttggcgga accgtcatgg gaaacaggtg     420

ctggatggaa atagtaaccc ctatgacatt ttcctaaagg acttggagcc gcccattgta     480

gccagatttg tccggttcat tccagtcacc gaccactcca tgaatgtgtg tatgagagtg     540

gagctttacg gctgtgtctg gctagatggc ttggtgtctt acaatgctcc agctgggcag     600

cagtttgtac tccctggagg ttccatcatt tatctgaatg attctgtcta tgatggagct     660

gttggataca gcatgacaga agggctaggc caattgaccg atggtgtgtc tggcctggac     720

gatttcaccc agacccatga ataccacgtg tggcccggct atgactatgt gggctggcgg     780

aacgagagtg ccaccaatgg ctacattgag atcatgtttg aatttgaccg catcaggaat     840

ttcactacca tgaaggtcca ctgcaacaac atgtttgcta aaggtgtgaa gatctttaag     900

gaggtacagt gctacttccg ctctgaagcc agtgagtggg aacctaatgc catttccttc     960

ccccttgtcc tggatgacgt caaccccagt gctcggtttg tcacggtgcc tctccaccac 1020
cgaatggcca gtgccatcaa gtgtcaatac cattttgcag atacctggat gatgttcagt 1080
gagatcacct tccaatcaga tgctgcaatg tacaacaact ctgaagccct gcccacctct 1140
cctatggcac ccacaaccta tgatccaatg cttaaagttg atgacagcaa cactcggatc 1200
ctgattggct gcttggtggc catcatcttt atcctcctgg ccatcattgt catcatcctc 1260
tggaggcagt tctggcagaa aatgctggag aaggcttctc ggaggatgct ggatgatgaa 1320
atgacagtca gcctttccct gccaagtgat tctagcatgt tcaacaataa ccgctcctca 1380
tcacctagtg aacaagggtc caactcgact tacgatcgca tctttcccct tcgccctgac 1440
taccaggagc catccaggct gatacgaaaa ctcccagaat ttgctccagg ggaggaggag 1500
tcaggctgca gcggtgttgt gaagccagtc cagcccagtg gccctgaggg ggtgccccac 1560
tatgcagagg ctgacatagt gaacctccaa ggagtgacag gaggcaacac atactcagtg 1620
cctgccgtca ccatggacct gctctcagga aaagatgtgg ctgtggagga gttccccagg 1680
aaactcctaa ctttcaaaga gaagctggga gaaggacagt ttggggaggt tcatctctgt 1740
gaagtggagg gaatggaaaa attcaaagac aaagattttg ccctagatgt cagtgccaac 1800
cagcctgtcc tggtggctgt gaaaatgctc cgagcagatg ccaacaagaa tgccaggaat 1860
gattttctta aggagataaa gatcatgtct cggctcaagg acccaaacat catccatcta 1920
ttagctgtgt gtatcactga tgaccctctc tgtatgatca ctgaatacat ggagaatgga 1980
gatctcaatc agtttctttc ccgccacgag ccccctaatt cttcctccag cgatgtacgc 2040
actgtcagtt acaccaatct gaagtttatg gctacccaaa ttgcctctgg catgaagtac 2100
ctttcctctc ttaattttgt tcaccgagat ctggccacac gaaactgttt agtgggtaag 2160
aactacacaa tcaagatagc tgactttgga atgagcagga acctgtacag tggtgactat 2220
taccggatcc agggccgggc agtgctccct atccgctgga tgtcttggga gagtatcttg 2280
ctgggcaagt tcactacagc aagtgatgtg tgggcctttg gggttacttt gtgggagact 2340
ttcacctttt gtcaagaaca gccctattcc cagctgtcag atgaacaggt tattgagaat 2400
actggagagt tcttccgaga ccaaggaggg cagacttacc tccctcaacc agccatttgt 2460
cctgactctg tgtataagct gatgctcagc tgctggagaa gagatacgaa gaaccgtccc 2520
tcattccaag aaatccacct tctgctcctt caacaaggcg acgagtga 2568

<210> 2
<211> 855
<212> PRT
<213> Homo sapiens

<400> 2

<210>  3
<211>  29
<212>  DNA
<213>  Artificial

<220>
<223>  primer

<400>  3
gaattcaaag atgtggctgt ggaggagtt                                          29

<210>  4
<211>  26
<212>  DNA
<213>  Artificial

<220>
<223>  primer

<400>  4
gctctagagc tcactcgtcg ccttgt                                             26